(19) Europäisches Patentamt
European Patent Office
Office européen des brevets

(11) EP 1 090 137 B1

(12) EUROPÄISCHE PATENTSCHRIFT

(45) Veröffentlichungstag und Bekanntmachung des Hinweises auf die Patenterteilung:
**03.04.2002 Patentblatt 2002/14**

(21) Anmeldenummer: **99929296.4**

(22) Anmeldetag: **22.06.1999**

(51) Int Cl.$^7$: **C12P 19/34**

(86) Internationale Anmeldenummer:
**PCT/EP99/04306**

(87) Internationale Veröffentlichungsnummer:
**WO 99/67413 (29.12.1999 Gazette 1999/52)**

(54) **HERSTELLUNG VON POLYMEREN AUS NUKLEOTIDISCHEN UND/ODER NICHT-NUKLEOTIDISCHEN BAUSTEINEN AUF ENZYMATISCHEM WEG**

PRODUCTION OF POLYMERS FROM NUCLEOTIDIC AND/OR NON-NUCLEOTIDIC ELEMENTS USING ENZYMATIC METHODS

PRODUCTION DE POLYMERES PAR VOIE ENZYMATIQUE A PARTIR D'ELEMENTS NUCLEOTIDIQUES ET/OU NON NUCLEOTIDIQUES

(84) Benannte Vertragsstaaten:
**AT BE CH DE DK ES FI FR GB IT LI LU NL PT SE**

(30) Priorität: **22.06.1998 DE 19827728**

(43) Veröffentlichungstag der Anmeldung:
**11.04.2001 Patentblatt 2001/15**

(73) Patentinhaber: **Larova Biochemie GmbH
14513 Teltow (DE)**

(72) Erfinder: **HAVLINA, Roxana, Maria
D-14513 Teltow (DE)**

(74) Vertreter: **Grund, Martin, Dr. et al
Dr. Volker Vossius
Patentanwaltskanzlei
Holbeinstrasse 5
81679 München (DE)**

(56) Entgegenhaltungen:
**WO-A-94/14972 WO-A-96/41809
US-A- 3 300 478**

- **MCCONNELL, TIMOTHY S.; CECH, THOMAS R.; HERSCHLAG, DANIEL: "Guanosine binding to the Tetrahymena ribozyme: Thermodynamic coupling with oligonucleotide binding" PROC. NATL. ACAD. SCI. U. S. A. , Bd. 90, Nr. 18, 1993, Seiten 8362-6, XP002120686**
- **CHEMICAL ABSTRACTS, vol. 110, no. 25, 1989 Columbus, Ohio, US; abstract no. 231999, Seite 686; Spalte links; XP002120690 & SHEVCHENKO, N. M.; SHALAMAI, A. S.; USENKO, L. S.: "Synthesis of modified triuridylates by the H-phosphonate method" BIOORG. KHIM., Bd. 14, Nr. 7, 1988, Seiten 976-8,**
- **HOLY, ANTONIN; GRUENBERGER, DEZIDER; SORM, FRANTISEK: " Effect of 5'-substitution on the template activity of oligonucleotides for the binding of valine and alanine tRNA to ribosomes" BIOCHIM. BIOPHYS. ACTA , Bd. 217, Nr. 2, 1970, Seiten 332-345, XP002120687**
- **KLEINWACHTER, V.; KOUDELKA, J.: "Luminescence properties of dinucleoside phosphates, some oligonucleotides, and polynucleotides" COLLECT. CZECH. CHEM. COMMUN., Bd. 37, Nr. 10, 1972, Seiten 3433-46, XP002120688**
- **VRATSKIKH, L. V.; KOMAROVA, N. I.; YAMKOVOY, V. I.: "Solid-phase synthesis of oligoribonucleotides using T4 RNA ligase and T4 polynucleotide kinase" BIOCHIMIE, Bd. 77, Nr. 4, 1995, Seiten 227-32, XP002120689**
- **PATENT ABSTRACTS OF JAPAN vol. 018, no. 086 (C-1165), 14. Februar 1994 (1994-02-14) & JP 05 292967 A (SHIMADZU CORP), 9. November 1993 (1993-11-09)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Herstellung natürlicher und/oder modifizierter Polymere mit einer definierten Sequenz aus nukleotidischen und/oder nicht-nukleotidischen Bausteinen auf enzymatischem Weg.

TECHNISCHER HINTERGRUND

Synthetische Oligonukleotide und Polynukleotide mit einer definierten Sequenz finden Anwendung in der Diagnostik und als Therapeutika, zum Beispiel als Antisense-Oligo-nukleotide, Ribozyme oder Aptamere.

Oligonukleotide können nach chemischen oder enzymatischen Methoden hergestellt werden. Die enzymatische Methode als Alternative für das herkömmliche chemische Verfahren gewinnt immer mehr an Bedeutung. Die bekannten enzymatischen Methoden verlaufen in 5'→3' Richtung.

Für die Herstellung von Oligonukleotiden auf enzymatischem Wege ist die Verwendung von folgenden Kettenverlängerungsenzymen möglich:

- einer Terminalen Transferase,
- einer Polynukleotid Phosphorylase,
- einer RNA Ligase oder
- einer DNA-Polymerase ( EP 0 491 739 B1).

Die Terminale Transferase, daß heißt die Terminale Desoxynukleotidyl-Transferase (EC 2.7.7.31) katalysiert die Addition von Desoxynukleotidtriphosphaten an das 3'-OH Ende einer einzel- oder doppelsträngigen DNA, in einer "Template"-unabhängigen Reaktion.

Die Polynukleotid Phosphorylase ( EC 2.7.7.8 ) katalysiert eine Reihe von Reaktionen, darunter die 5'→3' Polymerisation, an der Ribonukleosid 5'- diphosphate beteiligt sind.

Die RNA Ligase ( EC 6.5.1.3 ) katalysiert in Anwesenheit von ATP eine kovalente Verknüpfung zwischen einem 5'- Phosphatdonor und einem 3'-Hydroxylakzeptor.

Aus dem Reaktionsmechanismus der RNA Ligase ist zu erkennen, daß man bei den ablaufenden Reaktionsschritten zwischen einem ATP-abhängigen und einem ATP-unabhängigen Schritt unterscheiden kann. Zunächst bindet das Enzym ein ATP-Molekül an die ATP-Bindungsstelle, wobei im ersten Reaktionsschritt unter Freisetzung von $PP_i$ AMP kovalent mit dem Enzym verknüpft wird. Der Donor wird von seiner Enzym-Bindungsstelle über das 5'-terminale Nukleosid und dessen benachbarte Phosphatgruppen gebunden. Daraufhin kann im zweiten Reaktionsschritt die Aktivierung des Donors stattfinden, indem das enzymgebundene AMP auf die 5'-endständige Phosphatgruppe des Donors unter Bildung einer Phosphoanhydridbindung transferiert wird. Die Aktivierung des Donors läuft in einem ATP-abhängigen Schritt ab. Im dritten Reaktionsschritt wird die internukleosidische Bindung zwischen Akzeptor und Donor gebildet, wobei AMP freigesetzt wird. Die Ligation des aktivierten Donors A-5'pp5'-N mit dem Akzeptor ist ATP-unabhängig. Beide Reaktionsschritte können auch getrennt voneinander ablaufen.

Die enzymatische Synthese von Oligoribonukleotiden in Richtung 5'→3' ist bekannt. In der Synthese-Variante von Hyman /1/ wird der RNA Ligase der Akzeptor und ein adenyliertes Nukleosid 3',5'- bisphosphat mit einer Phosphatschutzgruppe in der 3'-Position als aktivierter Donor angeboten. Diese Reaktion wird durch das in Figur 1 gezeigte Schema erläutert.

Die Abkürzungen in Fig. 1 haben folgende Bedeutung:

AppNp ein aktivierter Donor, ein Nukleotid mit einer Phosphatschutzgruppe in der 3'-Position,
pNp ein Nukleotid mit einer Phosphatschutzgruppe in der 3'-Position,
pN ein Nukleotid ohne Schutzgruppe in der 3'-Position.

Die Hyman-Methode eignet sich gut für die Synthese von natürlichen und modifizierten Oligoribonukleotiden.

Für die Synthese von Oligodesoxyribonukleotiden schlägt Hyman

- die Synthese eines komplementären Oligoribonukleotides nach seiner Methode und
- die Umsetzung des Oligoribonukleotides in ein Oligodesoxyribonukleotid mit Hilfe der Reversen Transcriptase vor /1/2/.

Andere Verfahren beschreiben die Amplifikation eines Templates mit Hilfe der DNA Polymerase I /3/4/.

Diese bekannten Verfahren haben mehrere Nachteile.

Der erste Nachteil ist, daß man keine Oligonukleotide mit alternierender Sequenz, wie zum Beispiel mit natürlichen Phosphodiester- oder Thiophosphat-bindungen oder mit natürlichen Riboseresten oder 2'- Alkyl-substituierten

Riboseresten, herstellen kann, sondern nur eine einheitliche Art von Monomeren polymerisieren kann.

Der zweite Nachteil ist, daß man für die Synthese eines modifizierten Produktes, sofern man die gleiche Modifikation in allen Bausteinen wünscht, nicht die ungewöhnlich breite Substratspezifität der RNA Ligase für den Donor, sondern die sehr begrenzte Substratspezifität der Reversen Transkriptase anwenden muß.

Die RNA Ligase kann derzeit nur für die Synthese von natürlichen und modifizierten Oligoribonukleotiden eingesetzt werden. Der Grund dafür ist die sehr enge Spezifität der RNA Ligase für ein Oligoribonukleotid als Akzeptor.

Wenn das neu synthetisierte, wachsende Produkt der Akzeptor ist, kann man ein oder zwei Desoxyribonukleotidbausteine koppeln, danach ist das Verfahren nicht mehr praktikabel, da die RNA Ligase keine Oligodesoxyribonukleotide als Akzeptoren in der Polymerisationsreaktion in Richtung 5'→3' einbauen kann.

Bekannte Studien zur enzymatischen Synthese von Oligodesoxyribonukleotiden in Richtung 5'→3' beschränken sich auf komplizierte, für den technischen Maßstab nicht praktikable Verfahren /5/6/.

Biochimie (1995), 77, 227-32 beschreibt ein Verfahren zur Herstellung von Oligoribonukleotiden in Richtung 3' → 5' unter Verwendung einer RNA-Ligase.

## BESCHREIBUNG DER ERFINDUNG

Der vorliegenden Erfindung liegt die Aufgabe zugrunde, allgemein anwendbare neue enzymatische Verfahren unter Anwendung einer RNA Ligase zur Herstellung von verschiedenen Polymeren aus nukleotidischen und/oder nichtnukleotidischen Bausteinen ( Monomeren ), insbesondere zur Herstellung von natürlichen und/oder basenmodifizierten und/oder zuckermodifizierten und/oder an den Phosphatgruppen modifizierten Oligodesoxyribonukleotiden und/oder Oligoribonukleotiden und/oder nicht-nukleotidischen Polymeren, in Richtung 3'→5' mit einer definierten Sequenz der Bausteine bereitzustellen. Die erfindungsgemäß herstellbaren Polymere umfaßen auch Oligonukleotide und Polynukleotide in ihrer allgemeinsten Form. Eine weitere Aufgabe ist es, Trägermoleküle zur Durchführung der Verfahren bereitzustellen.

Die Lösung dieser Aufgaben ergeben sich aus der nachfolgenden Beschreibung und den Patentansprüchen.

Die Erfindung betrifft somit neue Verfahren zur Herstellung von Polymeren aus nukleotidischen und/oder nicht-nukleotidischen Bausteinen in Richtung 3'→5' unter Anwendung neuer synthetischer Träger, die gleichzeitig Trägermoleküle der erneut zu koppelnden natürlichen und/oder modifizierten Bausteine sind, mit Hilfe einer RNA Ligase sowie von Hilfsenzymen, wie einer RNase, einer Uracil DNA- Glycosylase, einer Apurinic Endonuklease und einer Polynukleotid Kinase.

Die vorliegende Erfindung umfaßt gemäß einer Ausführungsform die in Figur 2 schematisch erläuternden zyklischen enzymatischen Verfahren zur Herstellung von Polymeren:

Die Abkürzungen in Figur 2 haben folgende Bedeutung:

| | |
|---|---|
| p( dNp )$_m$ | Primer ( in diesem Fall Donor ) im Herstellungsverfahren 1, |
| p( rNp )$_m$ | Primer ( in diesem Fall Donor ) im Herstellungsverfahren 2, |
| ( rM )$_n$dN | neuer synthetischer Träger: Akzeptor im Herstellungsverfahren 1, |
| ( rM )$_n$dU-rN | neuer synthetischer Träger: Akzeptor im Herstellungsverfahren 2, |
| rM | ein Purin- und/oder Pyrimidinribonukleosid, |
| dU | ein Desoxyuridin, |
| dN | ein natürliches und/oder basen- und/oder zuckermodifiziertes Desoxyribonukleosid und/oder eine zur Ligation befähigte nichtnukleosidische Verbindungsgruppe, |
| rN | ein natürliches und/oder basen- und/oder zuckermodifiziertes Ribonukleosid und/oder eine zur Ligation befähigte nicht-nukleosidische Verbindungsgruppe, |
| p | eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe, |
| m | eine ganze Zahl von 1 oder mehr, |
| n | den Wert Null hat oder eine ganze Zahl von 1 oder mehr. |

Ausgehend von dem Primer ( in diesem Fall Donor ) der allgemeinen Formel p( dNp )$_m$ oder p( rNp )$_m$ mit der allgemeinen Strukturformel I

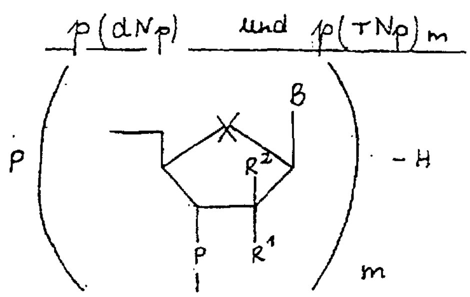

(I)

und dem erfindungsgemäß neuen synthetischen Träger ( in diesem Fall Akzeptor ) der allgemeinen Formel ( rM $)_n$dN mit der allgemeinen Strukturformel II

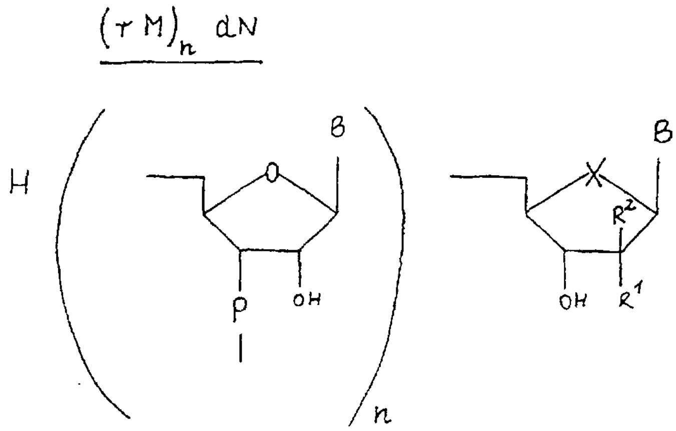

(II)

oder dem neuen Träger ( rM $)_n$dU-rN der allgemeinen Strukturformel III

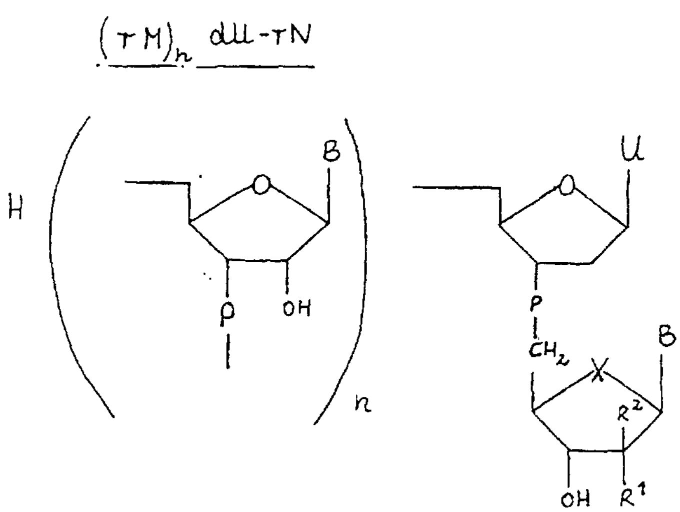

(III),

wobei in den Formeln die Abkürzungen folgende Bedeutung haben:

| | |
|---|---|
| m | eine ganze Zahl von 1 oder mehr, |
| n | den Wert Null hat oder eine ganze Zahl von 1 oder mehr, |
| B | eine natürliche und/oder modifizierte Purin- und/oder Pyrimidinbase, |

| | |
|---|---|
| U | ein Uracil, |
| $R^1$ und $R^2$ | H, OH, O-Alkyl, F, J, Br, Cl, Amino, Aminoalkyl, O-Alkoxyalkyl, Alkyl, |
| X | O, S, NH, $-NR^3$ ($R^3 = C_{1-20}$ Alkylrest), |
| p | eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe, |

wobei

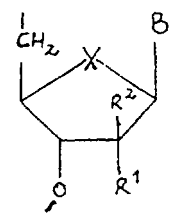

durch eine zur Ligation befähigte nicht-nukleosidische Verbindungsgruppe ersetzt sein kann,

wird zunächst im Ligationsschritt mit Hilfe einer RNA Ligase, in einer die Ligation ermöglichenden Menge, der Primer aktiviert und anschließend mit dem erfindungsgemäß verwendeten Träger ligiert. Bevorzugt wird der erfindungsgemäß verwendete Träger der allgemeinen Formel $( rM )_n dN$ oder $( rM )_n dU\text{-}rN$ im Überschuß zum Primer der allgemeinen Formel $p( dNp )_m$ oder $p( rNp )_m$ eingesetzt.

Aktivierung und Ligation können gemeinsam oder getrennt ablaufen.

Der Nukleosidbaustein dN oder rN des erfindungsgemäß verwendeten Trägers der allgemeinen Formel $( rM )_n dN$ oder $( rM )_n dU\text{-}rN$, welcher an die 5'- Position des Primers der allgemeinen Formel $p( dNp )_m$ oder $p( rNp )_m$ gekoppelt wird und Bestandteil der endgültig definierten Sequenz ist, besitzt keine Schutzgruppe in der 3'-Position.

Der erfindungsgemäß verwendete Träger der allgemeinen Formel $( rM )_n dN$ oder $( rM )_n dU\text{-}rN$ besitzt $( rM )_n$ oder $( rM )_n dU$ als Schutzgruppe in der 5'-Position, welche im Entschützungsschritt abgespalten wird.

Die genannte p-Gruppe in der 3'- Position des Primers der allgemeine Formel $p( dNp )_m$ oder $p( rNp )_m$ ist nicht beschränkend aufzufassen. Vorzugsweise kann darauf verzichtet werden.

Der erfindungsgemäß verwendete Träger der allgemeinen Formel $( rM )_n dN$ oder $( rM )_n dU\text{-}rN$ hat die höchste Affinität für RNA Ligasen in Gegenwart von Oligoribonukleotidbausteinen. Dadurch werden Ligationsausbeuten von etwa 100% erreicht. Die Struktur der erfindungsgemäß verwendeten Träger ist entscheidend für den Erfolg der 3'→ 5' Ligation in allen Verfahren der Erfindung.

Der Primer und der erfindungsgemäß verwendete Träger können als Metallsalze, vorzugsweise als Alkalimetallsalze, vorliegen.

Die Effizienz der Kopplungsreaktion ist weiterhin abhängig von verschiedenen Parametern:

1. Die RNA Ligasen besitzen ein breites pH-Spektrum im Bereich von etwa pH 7,0 bis pH 10,0. Die Ligation wird vorzugsweise bei einem pH von etwa 8,3 durchgeführt. Die Reaktion wird im gepufferten wässrigen Medium durchgeführt, vorzugsweise mit HEPES-NaOH oder HEPPS-NaOH. Der Einsatz von HEPPS-NaOH mit etwa 25mM bis 100mM im Vergleich zu HEPES-NaOH führt zu noch höheren Ausbeuten.
2. Die Ligation erfolgt in einem Temperaturbereich von etwa 4°C bis 40°C, bevorzugt bei etwa 37°C.
3. Die Ligationsreaktion verläuft nur in Gegenwart zweiwertiger Metallionen, vorzugs-weise mit etwa 10mM bis 20mM $Mn^{2+}$, und vorzugsweise in Anwesenheit von Dithiotreitol ( DTT ) mit etwa 3mM bis 20mM.
4. Die Ligationseffizienz wird vorzugsweise durch Zugabe einer die Ligationseffizienz erhöhenden Menge an Dimethylsulfoxid ( DMSO ), vorzugsweise 15 % ( v/v ), oder durch den Zusatz von PEG und Hexammincobaltchlorid, vorzugsweise 25% ( w/v ) Polyethylenglycol ( PEG 6000 ) und 1mM Hexammincobaltchlorid, erhöht.

Im Entschützungsschritt erfolgt in Gegenwart einer RNase der Verdau oder die alkalische Abspaltung der $( rM )_n$ Schutzgruppe bei Verwendung des Trägers der allgemeinen Formel $( rM )_n dN$. Hat n den Wert Null ist eine Abspaltung der $( rM )_n$ Schutzgruppe nicht notwendig, da das entstehende Polymer schon die korrekte definierte Sequenz besitzt. Bei Verwendung des Trägers der allgemeinen Formel $( rM )_n dU\text{-}rN$ katalysieren eine Uracil DNA- Glycosylase und eine Apurinic Endonuklease oder das Arbeiten im alkalischen Bereich die Abspaltung der $( rM )_n dU$ Schutzgruppe.

Im Phosphorylierungsschritt wird das gebildete Polymer an der 5'- Position mit Hilfe einer Polynukleotid

Kinase phosphoryliert oder thiophosphoryliert und der Zyklus kann erneut gestartet werden.

Die in Figur 2 schematisch dargestellten erfindungsgemäßen Herstellungsverfahren sind nicht beschränkend aufzufassen.

Dem Fachmann ist klar, daß auch Kombinationen der gezeigten Verfahren möglich sind. So können Primer der allgemeinen Formel p( dNp $)_m$ auch mit einem erfindungsgemäß verwendeten Träger der allgemeinen Formel ( rM $)_n$dU-rN, beziehungsweise ein Primer der allgemeinen Formel p( rNp $)_m$ kann auch mit einem erfindungsgemäß verwendeten Träger der allgemeinen Formel ( rM $)_n$dN zur Reaktion gebracht werden, um Polymere mit gewünschter, definierter Sequenz zu erhalten. Ferner kann ein Primer der allgemeinen Formel p( dNp $)_m$ auch Ribonukleotide bzw. ein Primer der allgemeinen Formel p( rNp $)_m$ auch Desoxyribonukleotide enthalten.

RNA Ligasen weisen eine ungewöhnlich breite Substratspezifität für den Donor auf. So sind neben den natürlichen Nukleotiden, wie den Mononukleosid 3',5'-bisphosphaten ( pNp ), eine Reihe von modifizierten Nukleotiden aktive Donoren für die RNA Ligasen. Modifikationen sind möglich sowohl an der Base, zum Beispiel *lin*-Benzoadenosin, 5- Fluoruridin, 5- Ioduridin, 2- Aminopurin, 2,6- Diaminopurin, $N^6$-Hexylaminoadenosin, am Zucker, beispielsweise 2'-O-Methylguanosin, 2'-O-Methyluridin, 2'-O-Methylcytidin und 2'-O-Methoxyethylnukleoside, als auch an den Phosphatgruppen, zum Beispiel 5'- Thiophosphoryl-pNp. Modifikationen am Zucker sind weiterhin neben 2'-O-Methylriboseanaloga auch 2'- Desoxyriboseanaloge oder Arabinoseanaloge. Modifikationen an den Phosphatgruppen sind Phosphorothioate und Phosphonate, wobei neben H-Phosphonaten auch Methylphosphonate Substrate für RNA Ligasen sind.

β- substituierte ADP-Derivate [ Ado- 5'PP- X ] sind bekanntlich ebenfalls aktive Substrate für die RNA Ligasen im Ligationsschritt. Nukleotidanaloge mit Modifikationen an der Base oder dem Zucker, Nukleotide mit einer α-N-glykosidischen Bindung, Phosphatester der Ribose, Riboflavine, Nicotinamidriboside, Pantetheine und Cyanoethanol können als aktivierte Donoren zum Akzeptor im Ligationsschritt dazugegeben werden, zum Beispiel adenyliertes Nikotinamid-Ribosid [ Ado- 5'PP5'- Nir ], adenyliertes 8-Brom-Adenosin [ Ado- 5'PP5'- ( 8- BrAdo ) ], Ado- 5'PP5'- riboflavin, adenyliertes 2'- Fluor-Adenosin [ Ado- 5'PP5'- ( 2'- FlAdo ) ] oder Ado- 5'PP6-cyanoethanol, wobei Ado für das Nukleosid Adenosin steht.

Infolge der Stereospezifität der RNA Ligasen ist die Herstellung von reinen $R_p$ Diastereoisomeren oder alternierenden $R_p$ / $S_p$ Isomeren erstmals im technischen Maßstab möglich.

Überraschenderweise wurde festgestellt, daß die RNA Ligasen ebenfalls in der Lage sind, Verknüpfungen zwischen einem 3'-Phosphatdonor und einem 5'-Hydroxylakzeptor zu bilden. Eine Aktivierung des Donors in der 3'-Position ist vorzugsweise bei hohen Konzentrationen von ATP und einem äquimolaren Verhältnis von Donor zu Akzeptor möglich. Auf Grund dieser Befunde wurden erfindungsgemäß die beiden folgenden Verfahren zur Herstellung von Polymeren entwickelt, die schematisch in Figur 3 erläutert sind.

Die Abkürzungen in Figur 3 haben folgende Bedeutung:

| | |
|---|---|
| ( dN $)_m$ | Primer ( in diesem Fall Akzeptor ) im Herstellungsverfahren 3, |
| ( rN $)_m$ | Primer ( in diesem Fall Akzeptor ) im Herstellungsverfahren 4, |
| p( rM $)_n$dNp | neuer synthetischer Träger: Donor im Herstellungsverfahren 3, |
| p( rM $)_n$dU-rNp | neuer synthetischer Träger: Donor im Herstellungsverfahren 4, |
| rM | ein Purin- und/oder Pyrimidinribonukleosid, |
| dU | ein Desoxyuridin, |
| dN | ein natürliches und/oder basen- und/oder zuckermodifiziertes Desoxyribonukleosid und/oder eine zur Ligation befähigte nicht-nukleosidische Verbindungsgruppe, |
| rN | ein natürliches und/oder basen- und/oder zuckermodifiziertes Ribonukleosid und/oder eine zur Ligation befähigte nicht-nukleosidische Verbindungsgruppe, |
| p | eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe, |
| m | eine ganze Zahl von 1 oder mehr, |
| n | den Wert Null hat oder eine ganze Zahl von 1 oder mehr. |

Ausgehend von dem erfindungsgemäß verwendeten Träger ( in diesem Fall Donor ) der allgemeinen Formel p( rM $)_n$dNp mit der allgemeinen Strukturformel IV

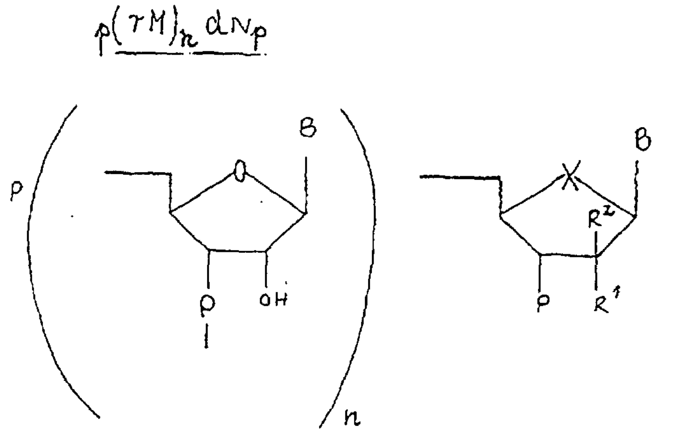

(IV)

oder dem neuen Träger der allgemeinen Formel p( rM )$_n$dU-rNp mit der allgemeinen Strukturformel V

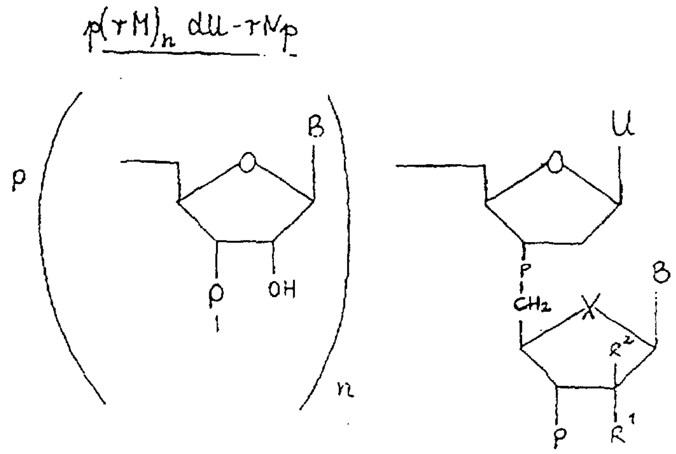

(V)

und dem Primer ( in diesem Fall Akzeptor ) der allgemeinen Formel ( dN )$_m$ bzw. ( rN )$_m$ mit der allgemeinen Strukturformel VI

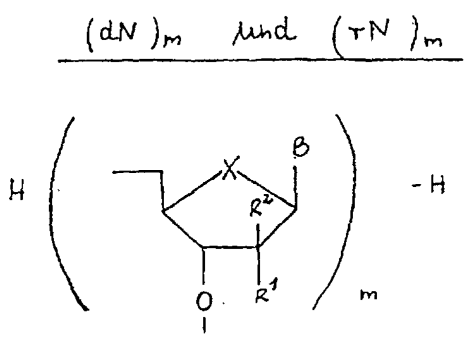

(VI)

wobei die Abkürzungen in den Formeln folgende Bedeutung haben:

| | |
|---|---|
| m | eine ganze Zahl von 1 oder mehr, |
| n | den Wert Null hat oder eine ganze Zahl von 1 oder mehr, |
| B | eine natürliche und/oder modifizierte Purin- und/oder Pyrimidinbase, |
| U | ein Uracil, |
| $R^1$ und $R^2$ | H, OH, O-Alkyl, F, J, Br, Cl, Amino, Aminoalkyl, O-Alkoxyalkyl, Alkyl, |

X O, S, NH, $-NR^3$ ($R^3 = C_{1-20}$ Alkylrest),

p eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe, wobei

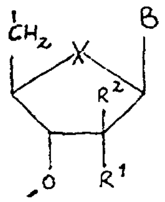

durch eine zur Ligation befähigte nicht-nukleosidische Verbindungsgruppe ersetzt sein kann,

wird zunächst im Ligationsschritt mit Hilfe einer RNA Ligase, in einer die Ligation ermöglichenden Menge, der erfindungsgemäß verwendete Träger aktiviert und anschließend mit dem Primer ligiert.

Aktivierung und Ligation können gemeinsam oder getrennt ablaufen.

Die p-Gruppe in der 3'- Position des erfindungsgemäßen Trägers der allgemeinen Formel p( rM $)_n$dNp oder p( rM $)_n$dU-rNp hat nicht die Funktion einer Schutzgruppe. Es erfolgt keine Abspaltung dieser Gruppe im Entschützungsschritt. Sie bleibt Bestandteil der endgültig definierten Sequenz.

Der erfindungsgemäß verwendete Träger der allgemeinen Formel p( rM $)_n$dNp oder p( rM $)_n$dU-rNp besitzt p ( rM $)_n$ oder p( rM $)_n$dU als Schutzgruppe in der 5'-Position, welche im Entschützungsschritt abgespalten wird.

Der Primer und der erfindungsgemäß verwendete Träger können als Metallsalze, vorzugsweise als Alkalimetallsalze, vorliegen.

Im Entschützungsschritt erfolgt in Gegenwart einer RNase der Verdau oder die alkalische Abspaltung der p ( rM $)_n$ Schutzgruppe bei Verwendung des Trägers der allgemeinen Formel p( rM $)_n$dNp. Hat n den Wert Null ist eine Abspaltung der p( rM $)_n$ Schutzguppe nicht notwendig, da das entstehende Polymer schon die korrekte definierte Sequenz besitzt. Bei Verwendung des Trägers der allgemeinen Formel p( rM $)_n$dU-rNp katalysieren eine Uracil DNA-Glycosylase und eine Apurinic Endonuklease oder das Arbeiten im alkalischen Bereich die Abspaltung der p( rM $)_n$dU Schutzgruppe.

Der Zyklus kann erneut gestartet werden.

Die in Figur 3 schematisch dargestellten erfindungsgemäßen Herstellungsverfahren sind nicht beschränkend aufzufassen.

Dem Fachmann ist klar, daß auch Kombinationen der gezeigten Verfahren möglich sind. So können Primer der allgemeinen Formel ( dN $)_m$ auch mit einem erfmdungsgemäß verwendeten Träger der allgemeinen Formel p ( rM $)_n$dU-rNp, beziehungsweise ein Primer der allgemeinen Formel ( rN $)_m$ kann auch mit einem erfindungsgemäß verwendeten Träger der allgemeinen Formel p( rM $)_n$dNp zur Reaktion gebracht werden, um Polymere mit gewünschter, definierter Sequenz zu erhalten. Ferner kann ein Primer der allgemeinen Formel ( dN $)_m$ auch Ribonukleotide bzw. ein Primer der allgemeinen Formel ( rN $)_m$ auch Desoxyribonukleotide enthalten.

BEISPIELE FÜR DIE SYNTHESE VON POLYMEREN

Die Beispiele erläutern die erfindungsgemäßen Verfahren. Sie sind in den Figuren 4 und 5 schematisch gezeigt. Die Beispiele sind nicht beschränkend aufzufassen.

Beispiel 1: Herstellungsverfahren 1

Im Ligationsschritt, der die Aktivierung und Ligation beinhaltet, wurde zunächst der Primer der allgemeinen Formel p( dNp $)_m$ in Gegenwart einer RNA Ligase mit ATP, 50mM HEPPS-NaOH als Puffer mit einem pH-Wert von etwa 8,3, 10 mM $MnCl_2$, 20 mM DTT, 10 µg/ ml Rinderserumalbumin ( BSA ) bei einer Temperatur von 30 bis 35°C aktiviert. Der Aktivierungsschritt erreichte innerhalb der ersten 3 Stunden eine Ausbeute von 96,8 % an aktiviertem Primer. Während des anschließenden Ligationsschrittes wurden die restlichen 3,2% komplett umgesetzt. Der aktivierte Primer wurde dabei mit dem erfindungsgemäß verwendeten Träger der allgemeinen Formel ( rM $)_n$dN unter den gleichen genannten Bedingungen, jedoch ohne ATP, ligiert. Die Ligationsreaktion verlief mit einer 100 %igen Umsetzung

des aktivierten Primers innerhalb von etwa 4,5 Stunden. Zusätzliche Informationen über die Identität des Ligationsproduktes gaben die Behandlung mit Alkalischer Phosphatase sowie die massenspektrometrische Messung.

Aktivierung und Ligation können gemeinsam oder getrennt unter den im Ligationsschritt genannten Reaktionsbedingungen stattfinden.

Mit der Erreichung einer 100% igen Umsetzung in kurzer Zeit wurden die schwierigsten technologischen Probleme: Aktivierung und Ligation, die bei der Realisierung eines technischen Verfahrens zur Herstellung von Polymeren eine Schlüsselrolle spielen, gelöst.

Im Entschützungsschritt erfolgte bei n > 1 die Abspaltung der $( rM )_n$ Schutzgruppe bei Verwendung des erfindungsgemäßen Trägers der allgemeinen Formel $( rM )_n dN$ in Gegenwart einer RNase unter den im Ligationsschritt genannten Bedingungen bei einer Temperatur von etwa 37°C oder die alkalische Abspaltung der $( rM )_n$ Schutzgruppe durch Erhöhung des pH-Wertes auf etwa 10,0 bis 11,5. Innerhalb von wenigen Minuten erfolgte die Abspaltung der Schutzgruppe der allgemeinen Formel $( rM )_n$.

Im Phosphorylierungsschritt wurde das Polymer unter sonst gleichen Bedingungen wie im Ligationsschritt mit Hilfe einer Polynukleotid Kinase bei einer Temperatur von etwa 37°C phosphoryliert oder thiophosphoryliert. Eine Polynukleotid Kinase katalysierte dabei den Transfer der endständigen Phosphatgruppe von ATP oder ATP-γ-S, wobei ATP-γ-S Adenosin 5'-O-( 3-thiotriphosphat) ist, auf die 5'-Hydroxylgruppe des Produktes. Die Reaktion verlief in 2-3 Minuten mit 100 % iger Ausbeute.

Der Zyklus konnte erneut gestartet werden bis ein Polymer gewünschter Länge und definierter Sequenz der Bausteine gebildet wurde. Die erneute Kopplung erfolgte unter den im Ligationsschritt genannten Bedingungen in 3,5 Stunden ebenfalls mit einer Ausbeute von etwa 100 %. Kürzere Reaktionszeiten konnten bei Verwendung höherer Enzymkonzentrationen realisiert werden. Die Reaktionsbedingungen waren für alle Reaktionsschritte vorzugsweise identisch und sind im Ligationsschritt genannt. Alle Reaktionsverläufe wurden mittels HPLC analysiert.

Als Primer der allgemeinen Formel $p( dNp )_m$ kann beispielsweise $p( dAp )_m$, $p( dCp )_m$, $p( dGp )_m$, $p( dTp )_m$ oder Kombinationen der Basen A,C,G und T verwendet werden sowie p(*lin*-Benzodesoxyadenosin 3'-Phosphat)$_m$, p (2-Aminodesoxyadenosin 3'-Phosphat)$_m$, p(2-Aminoalkyldesoxyadenosin 3'-Phosphat)$_m$, p(2-Heterocycloalkyldesoxyadenosin 3'-Phosphat)$_m$ p(5-Methyldesoxycytidin 3'-Phosphat)$_m$, p(5-Propinyldesoxycytidin 3'-Phosphat)$_m$, p (5-Ioddesoxyuridin 3'-Phosphat)$_m$, p(iso-Desoxycytidin 3'-Phosphat)$_m$, p(7-Propinyl-deaza-Desoxyguanosin 3'-Phosphat)$_m$, p(7-Propinyl-deaza-Desoxyadenosin 3'-Phosphat)$_m$, p(2'-O-Methylcytidin 3'-Phosphat)$_m$, p(2'-O-Methoxyethylguanosin 3'-Phosphat)$_m$, p(ara-G 3'-Phosphat)$_m$, p(2-Nitrophenylmethanol)$_m$, p(Nitrophenol)$_m$, p(Methoxyphenol)$_m$, p (2,6-Diaminopurin 3'-Phosphat)$_m$, ( 2'-Desoxyguanosin 3'-Phosphat 5'-Thiophosphat )$_m$, p(dC 3'-Thiophosphat)$_m$,und andere verwendet werden.

Als erfindungsgemäßer Träger der allgemeinen Formel $( rM )_n dN$ kann beispielsweise $(rG)_n$dC, $(rA)_n$2'-O-Methylcytidin, $(rA)_n$dG, $(rG)_n$2-Aminodesoxyadenosin, $(rC)_n$2-Amino-alkyldesoxyadenosin, $(rU)_n$2-Heterocycloalkyldesoxyadenosin, $(rA)_n$5-Methyl-desoxycytidin, $(rG)_n$5-Propinyldesoxycytidin, $(rC)_n$-2'-O-Methoxyethylguanosin, $(rG)_n$ $N^6$-Hexylaminodesoxy-adenosin, $(rU)_n$araG, $(rC)_n$ 2'-O-Methyladenosin, $(rC)_n$7-Propinyl-deaza-Desoxyguanosin, $(rG)_n$7-Propinyl-deaza-Desoxyadenosin, $(rG)_n$ 6-Cyanoethanol, $(rG)_n$5-Methyldesoxycytidin, $(rA)_n$2,6-Diaminopurin und andere verwendet werden.

Beispiel 2: Herstellungsverfahren 2

Im Ligationsschritt, der die Aktivierung und Ligation beinhaltet, wurde zunächst der Primer der allgemeinen Formel $p( rNp )_m$ in Gegenwart einer RNA Ligase mit ATP, 50mM HEPPS-NaOH als Puffer mit einem pH-Wert von etwa 8.3, 10 mM $MnCl_2$, 20 mM DTT, 10 µg/ ml Rinderserumalbumin ( BSA ) bei einer Temperatur von 30 bis 35°C aktiviert. Der Aktivierungsschritt erreichte innerhalb der ersten 3 Stunden eine Ausbeute von 96,8 % an aktiviertem Primer. Während des anschließenden Ligationsschrittes wurden die restlichen 3,2% komplett umgesetzt. Der aktivierte Primer wurde dabei mit dem erfindungsgemäß verwendeten Träger der allgemeinen Formel $( rM )_n dU\text{-}rN$ unter den gleichen genannten Bedingungen, jedoch ohne ATP, ligiert. Die Ligationsreaktion verlief mit 100 %iger Umsetzung des aktivierten Primers innerhalb von etwa 4,5 Stunden. Zusätzliche Informationen über die Identität des Ligationsproduktes gaben die Behandlung mit Alkalischer Phosphatase sowie die massenspektrometrische Messung.

Aktivierung und Ligation können gemeinsam oder getrennt unter den im Ligationsschritt genannten Reaktionsbedingungen ablaufen.

Mit der Erreichung einer 100% igen Umsetzung in kurzer Zeit wurden die schwierigsten technologischen Probleme: Aktivierung und Ligation, die bei der Realisierung eines technischen Verfahrens zur Herstellung von Polymeren eine Schlüsselrolle spielen, gelöst.

Im Entschützungsschritt erfolgte die Abspaltung der $( rM )_n dU$ Schutzgruppe bei Verwendung des erfindungsgemäßen Trägers der allgemeinen Formel $( rM )_n dU\text{-}rN$ in Gegenwart einer Uracil DNA- Glycosylase und einer Apurinic Endonuklease unter den im Ligationsschritt genannten Bedingungen bei einer Temperatur von etwa 37°C oder die alkalische Abspaltung der $( rM )_n dU$ Schutzgruppe durch Erhöhung des pH-Wertes auf etwa 10,0 bis 11,5. Innerhalb

von wenigen Minuten erfolgte die Abspaltung der Schutzgruppe der allgemeinen Formel ( rM )$_n$dU.

Im Phosphorylierungsschritt wurde das Polymer unter sonst gleichen Bedingungen wie im Ligationsschritt mit Hilfe einer Polynukleotid Kinase bei einer Temperatur von etwa 37°C phosphoryliert oder thiophosphoryliert. Eine Polynukleotid Kinase katalysierte dabei den Transfer der endständigen Phosphatgruppe von ATP oder ATP-γ-S, wobei ATP-γ-S Adenosin 5'-O-(3-thiotriphosphat) ist, auf die 5'- Hydroxylgruppe des Produktes. Die Reaktion verlief in 2-3 Minuten mit 100 %iger Ausbeute.

Der Zyklus konnte erneut gestartet werden bis ein Polymer gewünschter Länge und definierter Sequenz der Bausteine gebildet wurde. Die erneute Kopplung erfolgte unter den im Ligationsschritt genannten Bedingungen in 3,5 Stunden ebenfalls mit einer Ausbeute von etwa 100 %. Kürzere Reaktionszeiten wurden bei Verwendung höherer Enzymkonzentrationen realisiert. Die Reaktionsbedingungen waren für alle Reaktionsschritte vorzugsweise identisch und sind im Ligationsschritt genannt. Alle Reaktionsverläufe wurden mittels HPLC analysiert. Als Primer der allgemeinen Formel p( rNp )$_m$ kann beispielsweise p( rAp )$_m$, p( rCp )$_m$, ( rGp )$_m$, p( rTp )$_m$ oder Kombinationen der Basen A, C,G und T verwendet werden sowie p(*lin*-Benzoadenosin 3'-Phosphat)$_m$, p(2-Aminoadenosin 3'-Phosphat)$_m$, p(2-Aminoalkylyadenosin 3'-Phosphat)$_m$, p(2-Heterocycloalkyladenosin 3'-Phosphat)$_m$, p(5-Methylcytidin 3'-Phosphat)$_m$, p (5-Propinylcytidin 3'-Phosphat)$_m$, p(5-Ioduridin 3'-Phosphat)$_m$, p(iso-Cytidin 3'-Phosphat)$_m$, p(7-Propinyl-deaza-Guanosin 3'-Phosphat)$_m$, p(7-Propinyldeaza-Adenosin 3'-Phosphat)$_m$, p(2'-O-Methylcytidin 3'-Phosphat)$_m$, p(2'-O-Methoxyethylguanosin 3'-Phosphat)$_m$, p(ara-G 3'-Phosphat)$_m$, p(2-Nitrophenylmethanol)$_m$, p(Nitrophenol)$_m$, p(Methoxyphenol)$_m$, p(2,6-Diaminopurin 3'-Phosphat)$_m$, ( Guanosin 3'-Phosphat 5'-Thio-phosphat )$_m$, (Cytidin 3'-Thiophosphat 5'-Phosphat)$_m$,und andere verwendet werden.

Als erfindungsgemäßer Träger der allgemeinen Formel ( rM )$_n$dU-rN kann beispielsweise (rG)$_n$dU-rC, (rA)$_n$dU-2'-O-Methylcytidin, (rA)$_n$dU-rG, (rG)$_n$dU-2-Aminoadenosin, (rC)$_n$dU-2-Aminoalkyladenosin, (rU)$_n$dU-2-Heterocycloalkyladenosin, (rA)$_n$dU-5-Methylcytidin, (rG)$_n$dU-5-Propinylcytidin, (rC)$_n$dU-2'-O-Methoxyethylguanosin, (rG)$_n$dU-N$^6$-Hexylaminoadenosin, (rU)$_n$dU-araG, (rC)$_n$dU-2'-O-Methyladenosin, (rC)$_n$dU-7-Propinyl-deaza-Guanosin, (rG)$_n$dU-7-Propinyl-deaza-Adenosin, (rG)$_n$dU-6-Cyanoethanol, (rA)$_n$dU-2,6-Diaminopurin und andere verwendet werden.

Beispiel 3: Herstellungsverfahren 3

Im Ligationsschritt, der die Aktivierung und Ligation beinhaltet, wurde zunächst der erfindungsgemäß verwendete Träger der allgemeinen Formel p( rM )$_n$dNp in Gegenwart einer RNA Ligase mit ATP, 50mM HEPPS-NaOH als Puffer mit einem pH-Wert von etwa 8,3, 10 mM $MnCl_2$, 20 mM DTT, 10 µg/ ml Rinderserumalbumin ( BSA ) bei einer Temperatur von 30 bis 35°C aktiviert. Der Aktivierungsschritt erreichte innerhalb der ersten 3 Stunden eine Ausbeute von 96,8 % an aktiviertem Träger. Während des anschließenden Ligationsschrittes wurden die restlichen 3,2 % komplett umgesetzt. Der aktivierte Träger wurde dabei mit dem Primer der allgemeinen Formel ( dN )$_m$ unter den gleichen genannten Bedingungen, jedoch ohne ATP, ligiert. Die Ligationsreaktion verlief mit einer 100 %igen Umsetzung des aktivierten Primers innerhalb von etwa 4,5 Stunden. Zusätzliche Informationen über die Identität des Ligationsproduktes gaben die Behandlung mit Alkalischer Phosphatase sowie die massenspektrometrische Messung.

Aktivierung und Ligation können gemeinsam oder getrennt unter den im Ligationsschritt genannten Reaktionsbedingungen stattfinden.

Mit der Erreichung einer 100% igen Umsetzung in kurzer Zeit wurden die schwierigsten technologischen Probleme: Aktivierung und Ligation, die bei der Realisierung eines technischen Verfahrens zur Herstellung von Polymeren eine Schlüsselrolle spielen, gelöst.

Im Entschützungsschritt erfolgte bei n ≥ 1 die Abspaltung der p( rM )$_n$ Schutzgruppe bei Verwendung des erfindungsgemäßen Trägers der allgemeinen Formel p( rM )$_n$dNp in Gegenwart einer RNase unter den im Ligationsschritt genannten Bedingungen bei einer Temperatur von etwa 37°C oder die alkalische Abspaltung der p( rM )$_n$ Schutzgruppe durch Erhöhung des pH-Wertes auf etwa 10,0 bis 11,5. Innerhalb von wenigen Minuten erfolgte die Abspaltung der Schutzgruppe der allgemeinen Formel p( rM )$_n$.

Der Zyklus konnte erneut gestartet werden bis ein Polymer gewünschter Länge und definierter Sequenz der Bausteine gebildet wurde. Die erneute Kopplung erfolgte unter den im Ligationsschritt genannten Bedingungen in 3,5 Stunden ebenfalls mit einer Ausbeute von etwa 100 %. Kürzere Reaktionszeiten wurden bei Verwendung höherer Enzymkonzentrationen realisiert. Die Reaktionsbedingungen sind für alle Reaktionsschritte vorzugsweise identisch und sind im Ligationsschritt genannt. Alle Reaktionsverläufe wurden mittels HPLC analysiert.

Als Primer der allgemeinen Formel ( dN )$_m$ kann beispielsweise ( dA )$_m$, ( dC )$_m$, ( dG )$_m$, ( dT )$_m$ oder Kombinationen der Basen A,C,G und T verwendet werden sowie ( *lin*-Benzodesoxyadenosin )$_m$, (2-Aminodesoxyadenosin )$_m$, ( 2-Aminoalkyldesoxyadenosin )$_m$, (2-Heterocycloalkyldesoxyadenosin)$_m$, ( 5-Methyldesoxycytidin )$_m$, (5-Propinyldesoxycytidin)$_m$, (5-Ioddesoxyuridin)$_m$, (iso-Desoxycytidin)$_m$, (7-Propinyl-deaza-Desoxyguanosin)$_m$, (7-Propinyl-deaza-Desoxyadenosin)$_m$, (2'-O-Methylcytidin)$_m$, (2'-O-Methoxyethylguanosin)$_m$, (ara-Guanosin)$_m$, (2-Nitrophenylmethanol)$_m$, (Nitrophenol)$_m$, (Methoxyphenol)$_m$, (2,6-Diaminopurin)$_m$ und andere ver-

wendet werden.

Als erfindungsgemäßer Träger der allgemeinen Formel p( rM )$_n$dNp kann beispielsweise p(rG)$_n$dC 3'-Phosphat, p(rA)$_n$dG 3'-Phosphat, p(rA)$_n$2'-O-Methylcytidin 3'-Phosphat, p(rG)$_n$2-Aminodesoxyadenosin 3'-Phosphat, p (rC)$_n$2-Aminoalkyldesoxyadenosin 3'-Phosphat, p(rU)$_n$2-Heterocycloalkyldesoxyadenosin 3'-Phosphat, p(rA)$_n$5-Methyldesoxycytidin 3'-Phosphat, p(rG)$_n$5-Propinyldesoxycytidin 3'-Phosphat, p(rC)$_n$-2'-O-Methoxyethylguanosin 3'-Phosphat, p(rG)$_n$ N$^6$-Hexylaminodesoxyadenosin 3'-Phosphat, p(rU)$_n$araG 3'-Phosphat, p(rC)$_n$ 2'-O-Methyladenosin 3'-Phosphat, p(rC)$_n$7-Propinyl-deaza-Desoxyguanosin 3'-Phosphat, p(rG)$_n$7-Propinyl-deaza-Desoxyadenosin 3'-Phosphat, p(rG)$_n$ 6-Cyanoethanol, p(rG)$_n$5-Methyl-desoxycytidin 3'-Phosphat, p(rA)$_n$2,6-Diaminopurin 3'-Phosphat, p (rA)$_n$dC 3'-Thiophosphat, p(rA)$_n$dC 3'- Methylphosphonat, p(rG)$_n$dC 3'- H-Phosphonat, p(rG)$_n$dA 3'-Phosphoramidat, p(rU)$_n$dG 3'- Formacetal, p(C)$_n$dG 3'- Phosphorodithioat, p(rA)$_n$dC 3'-Boranophosphat, p(rG)$_n$dG 3'- Phosphotriester und andere verwendet werden.

Beispiel 4: Herstellungsverfahren 4

Im Ligationsschritt, der die Aktivierung und Ligation beinhaltet, wurde zunächst der erfindungsgemäß verwendete Träger der allgemeinen Formel p( rM )$_n$dU-rNp in Gegenwart einer RNA Ligase mit ATP, 50mM HEPPS-NaOH als Puffer mit einem pH-Wert von etwa 8,3, 10 mM $MnCl_2$, 20 mM DTT, 10 µg/ ml Rinderserumalbumin ( BSA ) bei einer Temperatur von 30 bis 35°C aktiviert. Der Aktivierungsschritt erreichte innerhalb der ersten 3 Stunden eine Ausbeute von 96,8 % an aktiviertem Träger. Während des anschließenden Ligationsschrittes wurden die restlichen 3,2 % komplett umgesetzt. Der aktivierte Träger wurde dabei mit dem Primer der allgemeinen Formel ( rN )$_m$ unter den gleichen genannten Bedingungen, jedoch ohne ATP, ligiert. Die Ligationsreaktion verlief mit einer 100 %igen Umsetzung des aktivierten Primers innerhalb von etwa 4,5 Stunden. Zusätzliche Informationen über die Identität des Ligationsproduktes gaben die Behandlung mit Alkalischer Phosphatase sowie die massenspektrometrische Messung.

Aktivierung und Ligation können gemeinsam oder getrennt unter den im Ligationsschritt genannten Reaktionsbedingungen stattfinden.

Mit der Erreichung einer 100% igen Umsetzung in kurzer Zeit wurden die schwierigsten technologischen Probleme: Aktivierung und Ligation, die bei der Realisierung eines technischen Verfahrens zur Herstellung von Polymeren eine Schlüsselrolle spielen, gelöst.

Im Entschützungsschritt erfolgte die Abspaltung der p( rM )$_n$dU Schutzgruppe bei Verwendung des erfindungsgemäßen Trägers der allgemeinen Formel p( rM )$_n$dU-rNp in Gegenwart einer Uracil DNA-Glycosylase und einer Apurinic Endonuklease unter den im Ligationsschritt genannten Bedingungen bei einer Temperatur von etwa 37°C oder die alkalische Abspaltung der p( rM )$_n$dU Schutzgruppe durch Erhöhung des pH-Wertes auf etwa 10,0 bis 11,5. Innerhalb von wenigen Minuten erfolgte die Abspaltung der p( rM )$_n$dU Schutzgruppe.

Der Zyklus konnte erneut gestartet werden bis ein Polymer gewünschter Länge und definierter Sequenz der Bausteine gebildet wurde. Die erneute Kopplung erfolgte unter den im Ligationsschritt genannten Bedingungen in 3,5 Stunden ebenfalls mit einer Ausbeute von etwa 100 %. Kürzere Reaktionszeiten wurden bei Verwendung höherer Enzymkonzentrationen realisiert. Die Reaktionsbedingungen sind für alle Reaktionsschritte vorzugsweise identisch und sind im Ligationsschritt genannt. Alle Reaktionsverläufe wurden mittels HPLC analysiert.

Als Primer der allgemeinen Formel ( rN )$_m$ kann beispielsweise ( rA )$_m$, ( rC )$_m$, ( rG )$_m$, ( rT )$_m$ oder Kombinationen der Basen A,C,G und T verwendet werden sowie (*lin*-Benzoadenosin)$_m$, (2-Aminoadenosin)$_m$, (2-Aminoalkyladenosin)$_m$, (2-Heterocycloalkyladenosin)$_m$, ( 5-Methylcytidin )$_m$, (5-Propinylcytidin)$_m$, (5-Ioduridin)$_m$, (iso-Cytidin)$_m$, (7-Propinyl-deaza-Guanosin)$_m$, (7-Propinyl-deaza-Adenosin)$_m$, (2'-O-Methylcytidin)$_m$, (2'-O-Methoxyethylguanosin)$_m$, (ara-Guanosin)$_m$, (2-Nitrophenylmethanol)$_m$, (Nitrophenol)$_m$, (Methoxyphenol)$_m$, (2,6-Diaminopurin)$_m$ und andere verwendet werden.

Als erfindungsgemäßer Träger der allgemeinen Formel p( rM )$_n$dU-rNp kann beispielsweise p(rG)$_n$dU-rC 3'-Phosphat, p(rA)$_n$dU-rG 3'-Phosphat, p(rA)$_n$dU-2'-O-Methylcytidin 3'-Phosphat, p(rG)$_n$dU-2-Aminoadenosin 3'-Phosphat, p(rC)$_n$dU-2-Amino-alkyladenosin 3'-Phosphat p(rU)$_n$dU-2-Heterocycloalkyladenosin 3'-Phosphat, p(rA)$_n$dU-5-Methylcytidin 3'-Phosphat, p(rG)$_n$dU-5-Propinylcytidin 3'-Phosphat, p(rC)$_n$dU-2'-O-Methoxyethylguanosin 3'-Phosphat, p(rG)$_n$dU-N$^6$-Hexylaminoadenosin 3'-Phosphat, p(rU)$_n$dU-araG 3'-Phosphat, p(rC)$_n$dU-2'-O-Methyladenosin 3'-Phosphat, p(rC)$_n$dU-7-Propinyl-deaza-Guanosin 3'-Phosphat, p(rG)$_n$dU-7-Propinyl-deaza-Adenosin 3'-Phosphat, p (rG)$_n$dU-6-Cyanoethanol, p(rA)$_n$dU-2,6-Diaminopurin 3'-Phosphat, p(rA)$_n$dU-rC 3'-Thiophosphat, p(rG)$_n$dU-rC 3'- Methylphosphonat, p(rG)$_n$dC 3'- H-Phosphonat, p(rG)$_n$dA 3'- Phosphoramidat, p(rU)$_n$dG 3'- Formacetal, p(C)$_n$dU-rG 3'-Phosphorodithioat, p(rA)$_n$dU-rC 3'- Boranophosphat, p(rG)$_n$dU-rG 3'- Phosphotriester und andere verwendet werden.

## ISOLIERUNG UND REINIGUNG VON POLYMEREN

Für die Isolierung und Reinigung von Polymergemischen können verschiedene chromatographische Verfahren eingesetzt werden. Hierzu gehören vorzugsweise die Hochdruckflüssigkeitschromatographie ( HPLC ) im analytischen Maßstab und die Anionenaustauscherchromatograhie im technischen Maßstab.

Beispiel 5: Isolierung und Reinigung von Polymeren im analytischen Maßstab

Für die Trennung von Polymergemischen in der Analytik wird vorzugsweise die RP-HPLC-Chromatographie angewandt. Die bei der RP-HPLC verwendeten Säulenmaterialien weisen einen geringen Durchmesser von 5 - 7 µm auf und erlauben somit in der Säule eine hohe Packungsdichte. Die RP-HPLC besitzt ein höheres Auflösungsvermögen und die Elutionszeit der zu trennenden Verbindungen ist stark verkürzt. Um die Retentionszeit und die Elutionsschärfe der Polymere zu verringern, wird die Polarität des eingesetzten Lösungsmittels, zum Beispiel Methanol oder Acetonitril, kontinuierlich verringert( Gradientenelution ). Als Reversed-Phase-Matrix wird chemisch modifiziertes Kieselgel ( Eurospher 100 C18, Fa. Knauer GmbH, Deutschland ) und als Elutionsmittel $KH_2PO_4$ 50 mM in Wasser und Methanol verwendet. Die Methanolkonzentration variiert, je nach Hydrophobizität des Polymers, zwischen 2,5 % und 25 %.

Beispiel 6: Reinigung und Isolierung von Polymeren im technischen Maßstab

Für die Trennung von Polymergemischen im technischen Maßstab ist die Säulenchromatographie an Anionenaustauschern, zum Beispiel Dowex-AG 1-X2, DEAE-Cellulose, DEAE-Sephadex, Source 15Q oder Source 30Q, geeignet. Die Elution geschieht in der Regel durch einen linearen Salzgradienten mit steigender Salzkonzentration. Da man die gewonnenen Polymere für weitere Reaktionen meist in einem salzfreien Zustand benötigt, werden zur Elution vorzugsweise ein Ammoniumbicarbonat-Puffer, ein Ammoniumacetat-Puffer oder ein Triethylammoniumbicarbonat- Puffer ( TEAB ) verwendet, die sich durch Lyophilisation oder Abdestillieren unter vermindertem Druck im Rotationsverdampfer bei niedriger Temperatur rückstandsfrei entfernen lassen.

AUTOMATISIERUNG

Die erfindungsgemäßen Verfahren eignen sich somit:

- für die Automatisierung im kleinen Maßstab zur Herstellung einer Vielfalt von Polymeren als auch
- im technischen Maßstab für die Herstellung von Wirkstoffen für Therapeutika.

Für die enzymatische Herstellung von Polymeren aus nukleotidischen und/oder nicht-nukleotidischen Bausteinen gibt es erfindungsgemäß mehrere Möglichkeiten.

Beispiel 7: Verfahren zur Herstellung von Polymeren im batch-Verfahren

Das erfmdungsgemäße Verfahren zur Herstellung von Polymeren ist im batch-Verfahren möglich. Alle Enzyme, wie die RNA Ligasen, RNasen, Uracil DNA- Glycosylasen, Apurinic Endonukleasen und die Polynukleotid Kinasen, werden nach jedem Reaktionsschritt inaktiviert. Die Inaktivierung einer RNase stellt dabei das größte Problem dar. Eine Inaktivierung ist jedoch unabdingbar, da eine aktive RNase im erneuten Kopplungsschritt die erfindungsgemäßen Trägermoleküle der Herstellungsverfahren 1 und 3 angreifen und zersetzen kann und demzufolge keine Ligationsreaktion mehr möglich ist. Die meisten RNasen sind bekanntlich sehr thermostabile Enzyme, welche nicht durch Hitze ( Temperaturen von 95°C ) inaktiviert werden können. Aus der Literatur sind eine Reihe von verschiedenen RNase Inhibitoren, wie hitzelabile RNase Inhibitor Proteine, Vanadyl Ribonukleosid Komplex als auch Bentonit, bekannt. Alle RNasen können hydrolysiert werden mit Hilfe von Proteasen, wie einer Proteinase K. Die Inkubation mit einer Proteinase K bei etwa 37°C und der anschließenden Hitzeinaktivierung ist eine effektive Möglichkeit zur Inaktivierung einer RNase. Die RNA Ligasen, Uracil DNA-Glycosylasen, Apurinic Endonukleasen und die Polynukleotid Kinasen werden ebenfalls durch Hitze inaktiviert.

Beispiel 8: Verfahren zur Herstellung von Polymeren mit immobilisierten Enzymen

Das erfindungsgemäße Verfahren zur Herstellung von Polymeren im technischen Maßstab kann auch mit immobilisierten Enzymen in Reaktoren in einem kontinuierlichen Verfahren durchgeführt werden. Die Enzyme werden nicht inaktiviert, sondern verbleiben durch die Immobilisierung an ein Trägermaterial in dem Reaktor für weitere Kopplungsschritte. Durch die Anwendung von Enzym-Membran-Reaktoren mit Membranen von 10 kDA ist ebenfalls die Nutzung der Enzyme für eine Reihe von Kopplungsschritten in einem kontinuierlichen Verfahren möglich.

Aufgrund der hohen Substratspezifität der RNA Ligasen ist es möglich, alle Generationen von nukleotidischen und nicht-nukleotidischen Polymeren mit einer definierten Sequenz und Länge in Richtung 3'→5' enzymatisch herzustellen.

LITERATUR

/1/ Hyman, E.D., US. PS 5,436,143 ( 1995 )
/2/ Hyman, E.D., US. PS 5,516,664 ( 1996 )
/3/ ISIS PHARMACEUTICALS, Inc., US.PS 5,576,302 ( 1996 )
/4/ Gen-Probe, Inc., US. PS 5,739,311 ( 1998 )
/5/ Moseman et al., Biochemistry, **19**, 635-642 ( 1980 )
/6/ Petric et al., Nucleic Acids Res., **19,** 585 ( 1990 )


**Patentansprüche**

1. Verfahren zur Herstellung von natürlichen und/oder modifizierten Polymeren mit einer definierten Sequenz aus nukleotidischen und/oder nicht-nukleotidischen Bausteinen, umfassend die folgenden Schritte:

   (a) Ligation eines Primers der allgemeinen Formel p( dNp $)_m$ oder p( rNp $)_m$, wobei die p-Gruppe in der 3'-Position des Primers gegebenenfalls fehlen kann, mit einem Träger der allgemeinen Formel ( rM $)_n$dN oder ( rM $)_n$dU-rN, in denen p, dN, rN, m, rM, n und dU die folgenden Bedeutungen haben:

   p eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe,
   dN ein natürliches und/oder basen- und/oder zuckermodifiziertes Desoxyribonukleosid und/oder eine zur Ligation befähigte nicht-nukleosidische Verbindungsgruppe,
   rN ein natürliches und/oder basen- und/oder zuckermodifiziertes Ribonukleosid und/oder eine zur Ligation befähigte nicht-nukleosidische Verbindungsgruppe,
   m eine ganze Zahl von 1 oder mehr,
   rM ein Purin- und/oder Pyrimidinribonukleosid,
   n eine ganze Zahl von 1 oder mehr,
   dU ein Desoxyuridin,

   wobei der Primer der allgemeinen Formel p( dNp $)_m$ oder p( rNp $)_m$ auch gemischte Reste von dN und/oder rN umfassen kann,
   mit Hilfe einer RNA Ligase in Gegenwart von zweiwertigen Metallkationen, wobei der Träger keine Schutzgruppe in der 3'- Position besitzt;

   (b) Entschützung des Ligationsproduktes bei Verwendung des Trägers der allgemeinen Formel ( rM $)_n$dN durch Abspaltung der ( rM $)_n$ Schutzgruppe in Gegenwart einer RNase bzw. Abspaltung der ( rM $)_n$dU Schutzgruppe bei Verwendung des Trägers der allgemeinen Formel ( rM $)_n$dU-rN mit Hilfe einer Uracil DNA-Glycosylase und einer Apurinic Endonuklease oder Abspaltung der genannten Schutzgruppen im alkalischen Bereich;

   (c) Transfer einer natürlichen und/oder modifizierten endständigen Gruppe, der vorstehend definierten allgemeinen Form p, auf die 5'- Hydroxylgruppe des Ligationsproduktes mit Hilfe einer Polynukleotid Kinase;

   (d) gegebenenfalls Wiederholung der Schritte (a) - (c) und;

   (e) gegebenenfalls Reinigung des im Schritt (c) erhaltenen Ligationsproduktes.

2. Verfahren zur Herstellung von natürlichen und/oder modifizierten Polymeren mit einer definierten Sequenz aus nukleotidischen und/oder nicht-nukleotidischen Bausteinen, umfassend die folgenden Schritte:

   (a) Ligation eines Primers der allgemeinen Formel ( dN $)_m$ oder ( rN $)_m$ mit einem Träger der allgemeinen Formel p( rM $)_n$dNp oder p( rM $)_n$dU-rNp, in denen dN, rN, m, p, rM, n und dU die folgenden Bedeutungen haben:

   dN ein natürliches und/oder basen- und/oder zuckermodifiziertes Desoxyribonukleosid und/oder eine zur

Ligation befähigte nicht-nukleosidische Verbindungsgruppe,

rN ein natürliches und/oder basen- und/oder zuckermodifiziertes Ribonukleosid und/oder eine zur Ligation befähigte nicht-nukleosidische Verbindungsgruppe,

m eine ganze Zahl von 1 oder mehr,

p eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe,

rM ein Purin- und/oder Pyrimidinribonukleosid,

n eine ganze Zahl von 1 oder mehr,

dU ein Desoxyuridin,

wobei der Primer der allgemeinen Formel ( dN )$_m$ oder ( rN )$_m$ auch gemischte Reste von dN und/oder rN umfassen kann,
mit Hilfe einer RNA Ligase in Gegenwart von zweiwertigen Metallkationen, wobei der Träger keine Schutzgruppe in der 3'- Position besitzt;

(b) Entschützung des Ligationsproduktes bei Verwendung des Trägers der allgemeinen Formel p( rM )$_n$dNp durch Abspaltung der p( rM )$_n$ Schutzgruppe in Gegenwart einer RNase bzw. Abspaltung der p( rM )$_n$dU Schutzgruppe bei Verwendung des Trägers der allgemeinen Formel p( rM )$_n$dU-rNp mit Hilfe einer Uracil DNA-Glycosylase und einer Apurinic Endonuklease oder Abspaltung der genannten Schutzgruppen im alkalischen Bereich;

(c) gegebenenfalls Wiederholung der Schritte (a) und (b) und;

(d) gegebenenfalls Reinigung des in Schritt (b) erhaltenen Ligationsproduktes.

**3.** Verfahren nach Anspruch 1 oder 2, **dadurch gekennzeichnet, daß** das Verfahren im batch-Verfahren durchgeführt wird, wobei nach jedem Schritt die Enzyme inaktiviert werden.

**4.** Verfahren nach Anspruch 1 oder 2, **dadurch gekennzeichnet, daß** das Verfahren mit immobilisierten Enzymen oder in Enzym-Membran-Reaktoren durchgeführt wird und die Enzyme nicht inaktiviert werden.

**5.** Verfahren nach Anspruch 1, wobei die Schritte a), b) und c) in einem Temperaturbereich von etwa 4 bis 40°C, vorzugsweise bei etwa 37°C, und in einem pH -Bereich von etwa 7,0 bis 10,0, vorzugsweise bei etwa 7,2 bis 8,5, insbesondere bei etwa 8,3, durchgeführt werden.

**6.** Verfahren nach Anspruch 2, wobei die Schritte a) und b) in einem Temperaturbereich von etwa 4 bis 40°C, vorzugsweise bei etwa 37°C, und in einem pH -Bereich von etwa 7,0 bis 10,0, vorzugsweise bei etwa 7,2 bis 8,5, insbesondere bei etwa 8,3, durchgeführt werden.

**7.** Verfahren nach Anspruch 1 oder 2, wobei das Reaktionsmedium als Puffer HEPPS-NaOH enthält.

**8.** Verfahren nach Anspruch 1 oder 2, wobei das Reaktionsmedium DMSO in einer die Ligationseffizienz steigernden Menge enthält.

**9.** Verwendung eines Trägermoleküls, ausgewählt aus der Gruppe von Molekülen der allgemeinen Formeln

( rM )$_n$dN

p( rM )$_n$dNp

( rM )$_n$dU-rN

p( rM )$_n$dU-rNp

wobei

rM ein Purin- und/oder Pyrimidinribonukleosid ist,

dN ein natürliches und/oder basen- und/oder zuckermodifiziertes Desoxyribonukleosid und/oder eine zur Ligation befähigte nicht-nukleosidische Verbindungsgruppe ist,

dU ein Desoxyuridin ist,

rN ein natürliches und/oder basen- und/oder zuckermodifiziertes Ribonukleosid und/oder eine zur Ligation befähigte nicht-nukleosidische Verbindungsgruppe ist,

p eine Phosphat- und/oder Phosphorothioat- und/oder Phosphonat-, wie vorzugsweise Methylphosphonat- und/oder H-Phosphonat-, und/oder Phosphoramidat- und/oder Fonnacetal- und/oder Phosphorodithioat- und/oder Boranophosphat- und/oder Phosphotriestergruppe ist,

n eine ganze Zahl von 1 oder mehr ist,

in einem Verfahren zur Herstellung von natürlichen und/oder modifizierten Polymeren gemäß einem der Ansprüche 1-8.

**10.** Verwendung gemäß Anspruch 9, wobei das Trägermolekül ausgewählt ist aus der Gruppe von Molekülen der allgemeinen Strukturformeln

$(rM)_n dN$

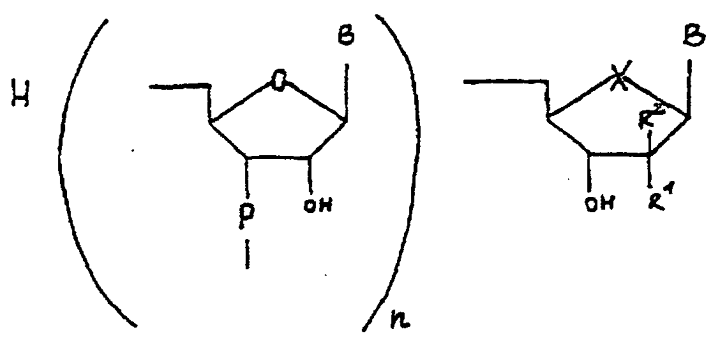

$(TM)_n$ dU-TN

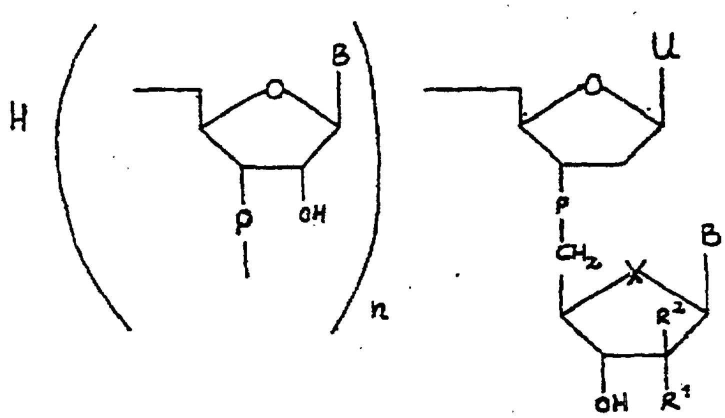

$p(TM)_n$ dNp

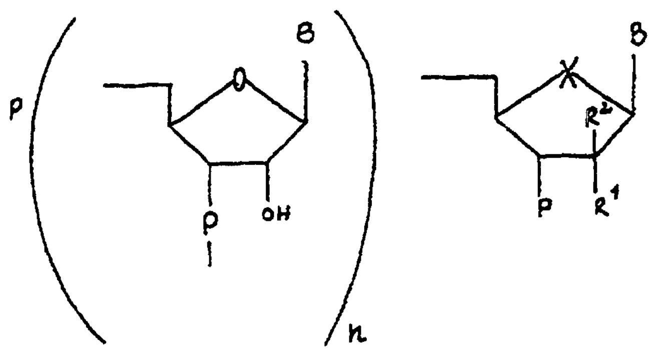

$p(TM)_n$ dU-TNp

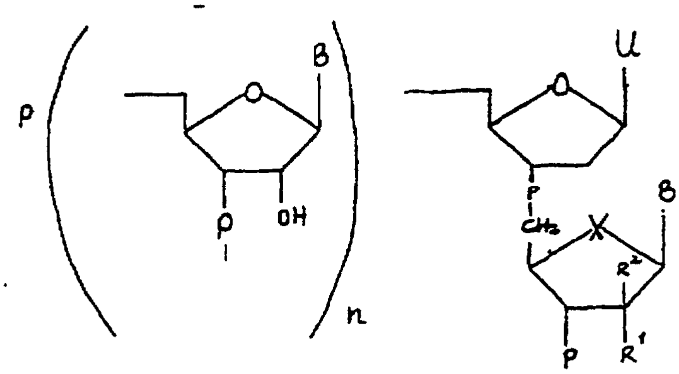

wobei

m eine ganze Zahl von 1 oder mehr ist,
n eine ganze Zahl von 1 oder mehr ist,
B eine natürliche und/oder modifizierte Purin- und/oder Pyrimidinbase ist,
U ein Uracil ist,

$R^1$ und $R^2$ H, OH, O-Alkyl, F, J, Br, Cl, Amino, Aminoalkyl, O-Alkoxyalkyl, Alkyl ist,

X O, S, NH, $-NR^3$ ($R^3 = C_{1-20}$ Alkylrest) ist,

p eine Phosphat- und/oder Phosphorothioat- und/oder Phosphonat-, wie vorzugsweise Methylphosphonat- und/oder H-Phosphonat-, und/oder Phosphoramidat- und/oder Formacetal- und/oder Phosphorodithioat- und/oder Boranophosphat- und/oder Phosphotriestergruppe ist,

wobei

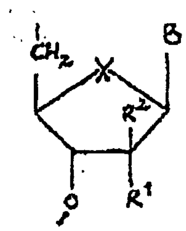

durch eine zur Ligation befähigte nicht-nukleosidische Verbindungsgruppe ersetzt sein kann.

## Claims

**1.** A method of preparing natural and/or modified polymers having a defined sequence of nucleotide and/or non-nucleotide monomers, comprising the steps of

(a) ligating a primer of the general formula $p(dNp)_m$ or $p(rNp)_m$, wherein the p-group in the 3'-position of the primer may be optionally absent, with a carrier of the general formula $(rM)_n dN$ or $(rM)_n dU\text{-}rN$ wherein p, dN, rN, m, rM, n and dU are:

p is a phosphate and/or phosphorothioate and/or phosphonate group such as preferably a methyl-phosphonate and/or H-phosphonate, and/or phosphoramidate and/or formylacetal and/or phosphorodithioate and/or boranephosphate and/or phosphotriester group

dN is a natural and/or base- and/or sugar-modified deoxyribonucleoside and/or a non-nucleoside ligation-competent group of compounds

rN is a natural and/or base- and/or sugar-modified ribonucleoside and/or a non-nucleoside ligation-competent group of compounds

m is an integer of 1 or more

rM is a purine and/or pyrimidine ribonucleoside

n is an integer of 1 or more.

dU is a deoxyuridine

wherein the primer of the general formula $p(dNp)_m$ or $p(rNp)_m$ may comprise mixed residues of dN and/or rN, with an RNA ligase in the presence of bivalent metal cations, the carrier having no protecting group in the 3' position;

(b) deprotecting the ligation product when using a carrier of the general formula $(rM)_n dN$ by cleavage of the $(rM)_n$ protecting group in the presence of an RNase or by cleavage of the $(rM)_n dU$ protecting group when using a carrier of the general formula $(rM)_n dU\text{-}rN$ by an uracil DNA glycosylase and an apurinic endonuclease or cleavage of said protecting groups in alkaline milieu;

(c) transfer of a natural and/or modified terminal group of the above defined general formula p on the 5' hydroxy group of the ligation product by a polynucleotide kinase;

(d) optionally repeating steps (a) to (c) and

(e) optionally purifying the ligation products obtained in step (c).

**2.** A method of preparing natural and/or modified polymers having a defined sequence of nucleotide and/or non-nucleotide monomers, comprising the steps of

a) ligating a primer of the general formula $(dN)_m$ or $(rN)_m$ with a carrier of the general formula $p(rM)_n dNp$ or $p(rM)_n dU\text{-}rNp$ wherein dN, rN, m, p, rM, n and dU are:

dN is a natural and/or base- and/or sugar-modified deoxyribonucleoside and/or a non-nucleoside ligation-competent group of compounds
rN is a natural and/or base- and/or sugar-modified ribonucleoside and/or a non-nucleoside ligation-competent group of compounds
m is an integer of 1 or more
p is a phosphate and/or phosphorothioate and/or phosphonate group such as preferably a methyl-phosphonate and/or H-phosphonate, and/or phosphoramidate and/or formylacetal and/or phosphorodithioate and/or boranephosphate and/or phosphotriester group
rM is a purine and/or pyrimidine ribonucleoside
n is an integer of 1 or more.
dU is a deoxyuridine

wherein the primer of the general formula $(dN)_m$ or $(rN)_m$ may comprise mixed residues of dN and/or rN, with an RNA ligase in the presence of bivalent metal cations, the carrier having no protecting group in the 3' position;
b) deprotecting the ligation product when using a carrier of the general formula $p(rM)_ndNp$ by cleavage of the $p(rM)_n$ protecting group in the presence of an RNase or by cleavage of the $p(rM)_ndU$ protecting group when using a carrier of the general formula $p(rM)_ndU\text{-}rNp$ by an uracil DNA glycosylase and an apurinic endonuclease or cleavage of said protecting groups in alkaline milieu;
c) optionally repeating steps (a) and (b) and
d) optionally purifying the ligation product obtained in step (b).

**3.** Method according to claim 1 or 2 wherein the method is carried out in a batch process and the enzymes are deactivated after each step.

**4.** Method according to claim 1 or 2 wherein the method is carried out with immobilized enzymes or in enzyme membrane reactors and the enzymes are not deactivated.

**5.** Method according to claim 1 wherein the steps (a), (b) and (c) are carried out in a temperature range from about 4 to 40°C, preferably at about 37°C and in a pH range of about 7.0 to 10.0, preferably of about 7.2 to about 8.5, most preferably at about 8.3.

**6.** Method according to claim 2 wherein the steps (a) and (b) are carried out in a temperature range from about 4 to 40°C, preferably at about 37°C and in a pH range of about 7.0 to 10.0, preferably of about 7.2 to about 8.5, most preferably at about 8.3.

**7.** Method according to claim 1 or 2 wherein the reaction medium is buffered with HEPPS-NaOH.

**8.** Method according to claim I or 2 wherein the reaction medium contains DMSO in a ligation efficiency increasing amount.

**9.** Use of a carrier molecule selected from the group consisting of molecules of the general formula

$$(rM)_ndN$$

$$p(rM)_ndNp$$

$$(rM)_ndU\text{-}rN$$

$$p(rM)_ndU\text{-}rNp$$

wherein

rM is a purine and/or pyrimidine ribonucleoside
dN is a natural and/or base- and/or sugar-modified deoxyribonucleoside and/or a non-nucleoside ligation-competent group of compounds

- dU is a deoxyuridine
- rN is a natural and/or base- and/or sugar-modified ribonucleoside and/or a non-nucleoside ligation-competent group of compounds
- p is a phosphate and/or phosphorothioate and/or phosphonate group such as preferably a methyl-phosphonate and/or H-phosphonate, and/or phosphoramidate and/or formylacetal and/or phosphorodithioate and/or boranephosphate and/or phosphotriester group
- n is an integer of 1 or more

in a method for the production of natural and/or modified polymers according to any one of claims 1 to 8.

**10.** Use according to claim 9, wherein the carrier molecule is selected from the group consisting of molecules of the general formula

$(rN)_n\ dN$

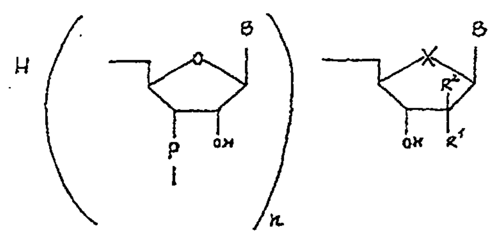

$(rN)_n\ dU\text{-}rN$

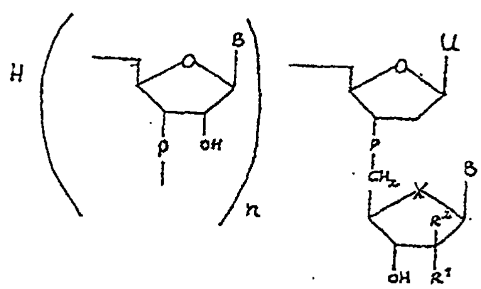

$p(rN)_n\ dN_p$

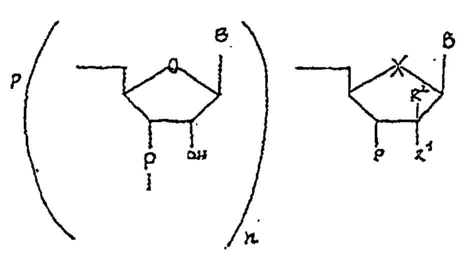

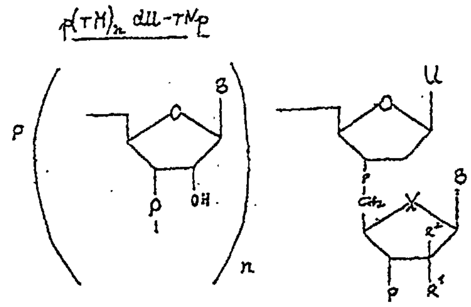

wherein

| | |
|---|---|
| m | is an integer of 1 or more |
| n | is an integer of 1 or more |
| B | is a natural and/or modified purine and/or pyrimidine base |
| U | is an uracil |
| $R^1$ and $R^2$ | is H, OH, O-alkyl, F, J, Br, Cl, amino, aminoalkyl. O-alkoxyalkyl, or alkyl |
| X | is O, S, NH, $-NR^3$ ($R^3 = C_{1-20}$ alkyl) |
| p | is a phosphate and/or phosphorothioate and/or phosphonate group such as preferably a methylphosphonate and/or H-phosphonate, and/or phosphoramidate and/or formylacetal and/or phosphorodithioate and/or boranephosphate and/or phosphotriester group |

and wherein

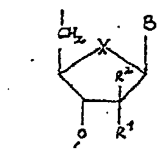

may be replaced by a non-nucleoside ligation-competent group of compounds.

## Revendications

**1.** Procédé pour la préparation de polymères naturels et/ou modifiés ayant une séquence définie de monomères nucléotidiques et/ou non-nucléotidiques, comportant les étapes suivantes:

(a) ligature d'une amorce de formule générale $p(dNp)_m$ ou $p(rNp)_m$ tandis que le groupe p en position 3' peut éventuellement être absent, avec un support de formule générale $(rM)_n dN$ ou $(rM)_n dU$-rN, dans lesquelles p, dN, rN, m, rM, n et dU ont les significations suivantes:

| | |
|---|---|
| p | un groupe phosphate et/ou phosphorothioate et/ou phosphonate, tel que de préférence méthylphosphonate et/ou H-phosphonate, et/ou phosphoramidate et/ou formacétal et/ou phosphorodithioate et/ou boranophosphate et/ou phosphotriester, |
| dN | un désoxyribonucléoside naturel et/ou modifié quant aux bases et/ou quant aux sucres, et/ou un groupe de composés non-nucléosidique, rendu apte pour la ligature, |
| rN | un ribonucléoside naturel et/ou modifié quant aux bases et/ou quant aux sucres, et/ou un groupe de composés non-nucléosidique, rendu apte pour la ligature, |
| m | un nombre entier de 1 ou plus, |
| rM | un ribonucléoside de purine et/ou pyrimidine, |
| n | un nombre entier de 1 ou plus, |
| dU | une désoxyuridine, |

tandis que l'amorce de formule générale p( dNp $)_m$ ou p( rNp $)_m$ peut également comporter des restes mixtes de dN et/ou rN, avec l'aide d'une ARN ligase en présence de cations de métaux divalents, tandis que le support ne possède aucun groupe protecteur sur la position 3';

(b) déprotection du produit de ligature avec utilisation du support de formule générale ( rM $)_n$dN par séparation du groupe protecteur ( rM $)_n$ en présence d'une ribonucléase ou séparation du groupe protecteur ( rM $)_n$dU avec utilisation du support de formule générale ( rM $)_n$dU-rN avec l'aide d'une uracile ADN-glycosylase et d'une endonucléase apurinique ou séparation des groupes protecteurs indiqués dans le domaine alcalin;

(c) transfer d'un groupe terminal naturel et/ou modifié, ayant la forme générale p définie plus haut, sur le groupe 5'-hydroxyle du produit de ligature avec l'aide d'une polynucléotide kinase;

(d) éventuellement répétition des étapes (a) - (c) et;

(e) éventuellement purification du produit de ligature obtenu dans l'étape (c).

**2.** Procédé pour la préparation de polymères naturels et/ou modifiés ayant une séquence définie de monomères nucléotidiques et/ou non-nucléotidiques, comportant les étapes suivantes:

(a) ligature d'une amorce de formule générale ( dN $)_m$ ou ( rN $)_m$ avec un support de formule générale p ( rM $)_n$dNp ou p( rM $)_n$dU-rNp, dans lesquelles dN, rN, m, p, rM, n et dU ont les significations suivantes:

dN un désoxyribonucléoside naturel et/ou modifié quant aux bases et/ou quant aux sucres et/ou un groupe de composés non-nucléosidique, rendu apte pour la ligature,

rN un ribonucléoside naturel et/ou modifié quant aux bases et/ou quant aux sucres, et/ou un groupe de composés non-nucléosidique, rendu apte pour la ligature,

m un nombre entier de 1 ou plus,

p un groupe phosphate et/ou phosphorothioate et/ou phosphonate, tel que de préférence méthylphosphonate et/ou H-phosphonate, et/ou phosphoramidate et/ou formacétal et/ou phosphorodithioate et/ou boranophosphate et/ou phosphotriester,

rM un ribonucléoside de purine et/ou pyrimidine,

n un nombre entier de 1 ou plus,

dU une désoxyuridine,

tandis que l'amorce de formule générale ( dN $)_m$ ou ( rN $)_m$ peut également comporter des restes mixtes de dN et/ou rN, avec l'aide d'une ARN ligase en présence de cations de métaux divalents, tandis que le support ne possède aucun groupe protecteur sur la position 3';

(b) déprotection du produit de ligature avec utilisation du support de formule générale p( rM $)_n$dNp par séparation du groupe protecteur p( rM $)_n$ en présence d'une ribonucléase ou séparation du groupe protecteur p ( rM $)_n$dU avec utilisation du support de formule générale p( rM $)_n$dU-rNp avec l'aide d'une uracile ADN-glycosylase et d'une endonucléase apurinique ou séparation du groupe protecteur indiqué dans le domaine alcalin;

(c) éventuellement répétition des étapes (a) et (b) et;

(d) éventuellement purification du produit de ligature obtenu dans l'étape (b).

**3.** Procédé selon la revendication 1 ou 2, **caractérisé en ce que** le procédé est mis en oeuvre en discontinu, tandis que l'enzyme est inactivée après chaque étape.

**4.** Procédé selon la revendication 1 ou 2, **caractérisé en ce que** le procédé est mis en oeuvre avec des enzymes immobilisées ou dans des réacteurs à membrane avec enzyme et les enzymes ne sont par inactivées.

**5.** Procédé selon la revendication 1, dans lequel les étapes a), b) et c) sont effectuées dans un intervalle de température d'environ 4 à 40°C, de préférence à environ 37°C, et dans un intervalle de pH d'environ 7,0 à 10,0, de préférence d'environ 7,2 à 8,5, en particulier à environ 8,3.

**6.** Procédé selon la revendication 2, dans lequel les étapes a) et b) sont effectuées dans un intervalle de température d'environ 4 à 40°C, de préférence à environ 37°C, et dans un intervalle de pH d'environ 7,0 à 10,0, de préférence d'environ 7,2 à 8,5, en particulier à environ 8,3.

**7.** Procédé selon la revendication 1 ou 2, dans lequel le milieu de réaction contient comme tampon HEPPS-NaOH.

**8.** Procédé selon la revendication 1 ou 2, dans lequel le milieu de réaction contient du DMSO dans une quantité

augmentant l'efficacité de ligature.

**9.** Utilisation d'une molécule support, choisie dans le groupe des molécules de formules générales

$$(\mathrm{rM})_n\mathrm{dN}$$

$$\mathrm{p}(\mathrm{rM})_n\mathrm{dNp}$$

$$(\mathrm{rM})_n\mathrm{dU\text{-}rN}$$

$$\mathrm{p}(\mathrm{rM})_n\mathrm{dU\text{-}rNp}$$

où

rM est un ribonucléoside de purine et/ou de pyrimidine,
dN est un désoxyribonucléside naturel et/ou modifié quant aux bases et/ou quant aux sucres, et/ou un groupe de composés non-nucléosidique, rendu apte pour la ligature,
dU est une désoxyuridine,
rN est un ribonucléoside naturel et/ou modifié quant aux bases et/ou quant aux sucres et/ou un groupe de composés non-nucléosidique, rendu apte pour la ligature,
P est un groupe phosphate et/ou phosphorothioate et/ou phosphonate, tel que de préférence méthylphosphonate et/ou H-phosphonate, et/ou phosphoramidate et/ou formacétal et/ou phosphorodithioate et/ou boranophosphate et/ou phosphotriester,
n est un nombre entier de 1 ou plus,

dans un procédé pour la préparation de polymères naturels et/ou modifiés selon l'une des revendications 1-8.

**10.** Utilisation selon la revendication 9, dans laquelle la molécule support est choisie dans le groupe des molécules de formules structurales générales

$(rM)_n\ dN$

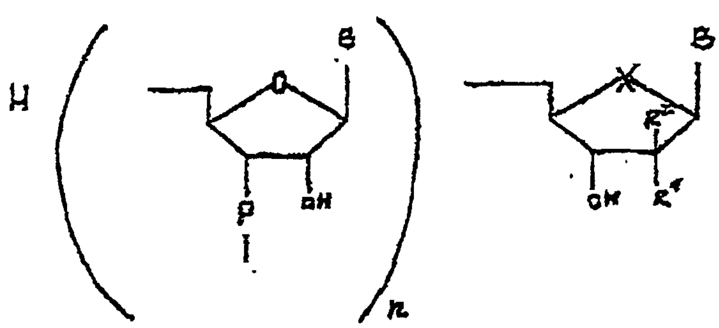

$(TM)_n$ dU-TN

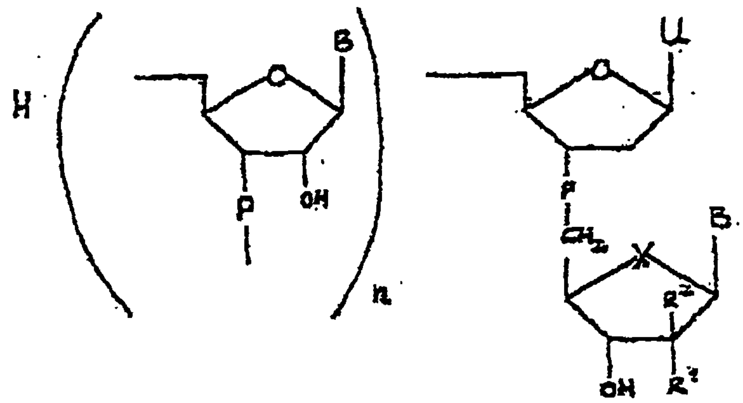

$p(TM)_n$ dNp

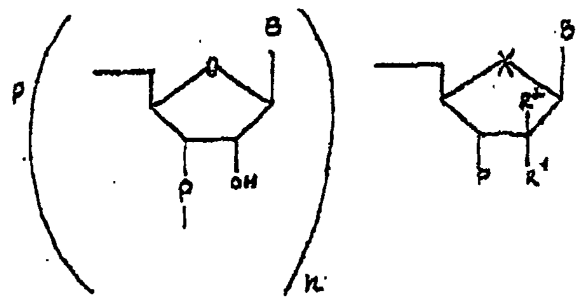

$p(TM)_n$ dU-TNp

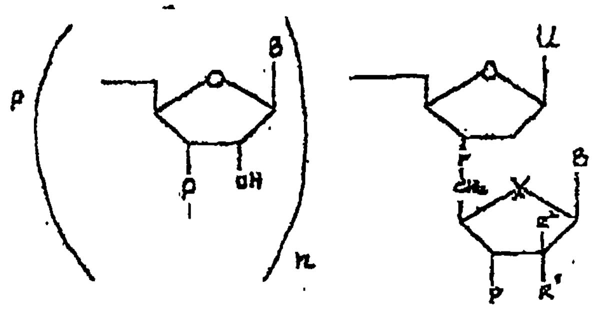

où

- m est un nombre entier de 1 ou plus,
- n est un nombre entier de 1 ou plus,
- B est une base purine et/ou pyrimidine naturelle et/ou modifiée,
- U est un uracile,
- $R^1$ et $R^2$ est H, OH, O-alkyle, F, I, Br, Cl, amino, aminoalkyle, O-alcoxyalkyle, alkyle,
- X est O, S, NH, $-NR^3$ ($R^3$ = reste alkyle en $C_{1-20}$),
- p est un groupe phosphate et/ou phosphorothioate et/ou phosphonate, tel que de préférence méthylphosphonate et/ou H-phosphonate, et/ou phosphoramidate et/ou formacétal et/ou phosphorodithioate

et/ou boranophosphate et/ou phosphotriester,

tandis que

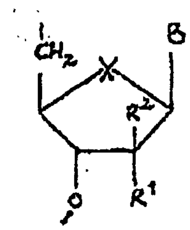

peut être remplacé par un groupe de composés non-nucléosidique rendu apte pour la ligature.

Verfahren zur Herstellung von Oligonukleotiden in Richtung 5'→3' nach Hyman

**Akzeptor + AppNp**

*RNA Ligase*
*5'-Nukleotidase*

**Akzeptor-pNp + AppNp + Adenosin + $HPO_4$**

*Phosphodiesterase I*
*5'-Nukleotidase*

**Akzeptor-pNp + 3',5'-NDP + Adenosin + $HPO_4$**

*Alkalische Phosphatase*

**Akzeptor-pN + $HPO_4$ + Nukleoside**

wiederholte Zugabe von AppNp
für den nächsten Kopplungsschritt

Figur 1

1. Verfahren zur Herstellung von Polymeren aus desoxyribonukleotidischen und/oder nicht-nukleotidischen Bausteinen

2. Verfahren zur Herstellung von Polymeren aus ribonukleotidischen und/oder nicht-nukleotidischen Bausteinen

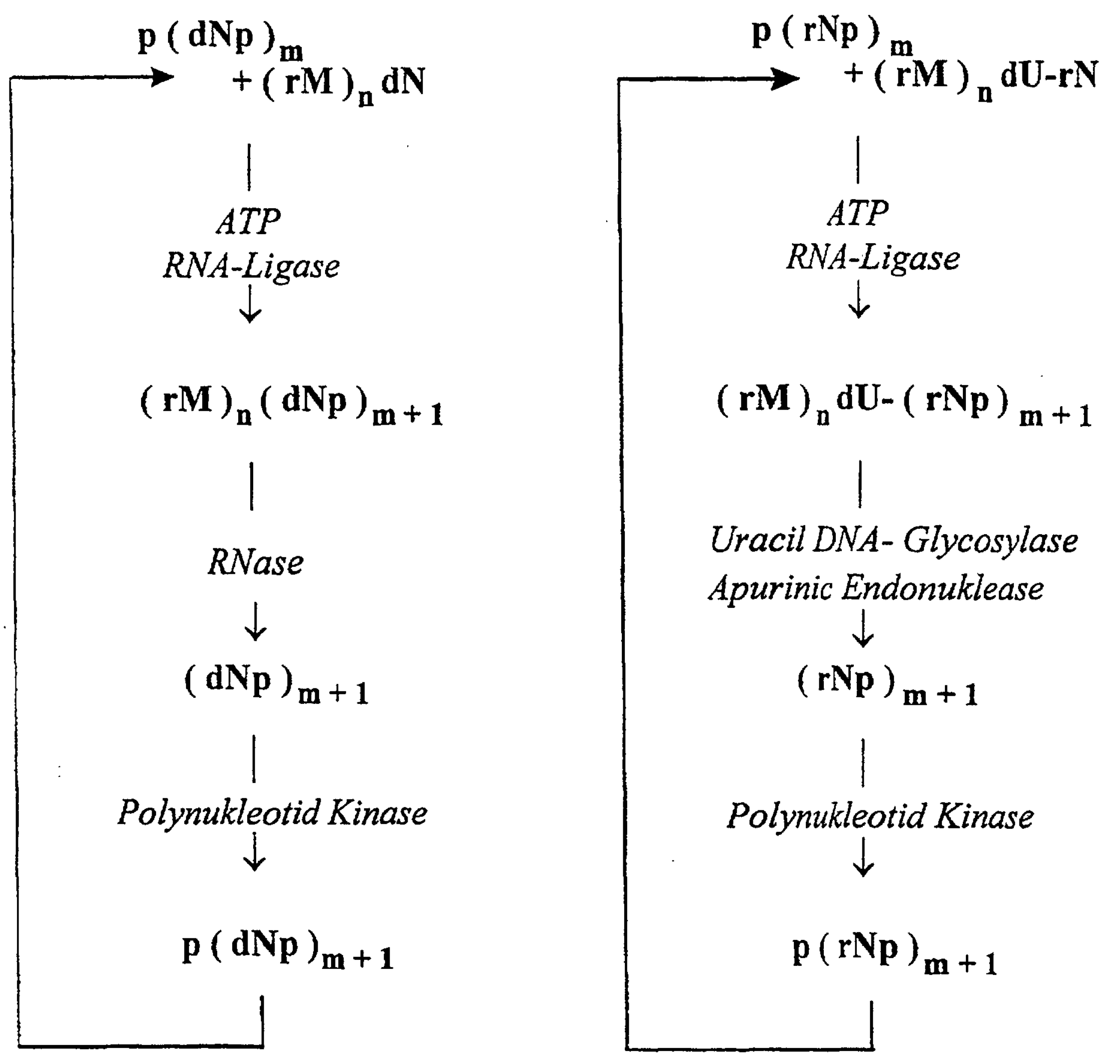


Figur 2

3. Verfahren zur Herstellung von Polymeren aus desoxyribonukleotidischen und/oder nicht-nukleotidischen Bausteinen

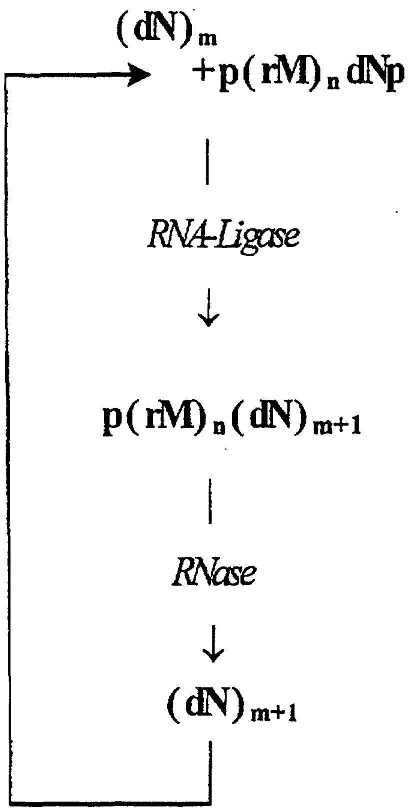


4. Verfahren zur Herstellung von Polymeren aus ribonukleotidischen und/oder nicht-nukleotidischen Bausteinen

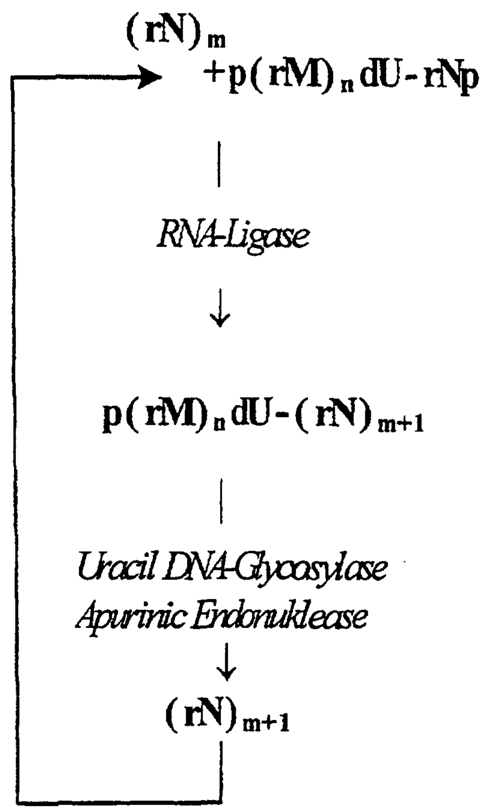


Figur 3

1. und 2. Verfahren zur Herstellung von Polymeren

Akzeptor ( Träger )

Primer ( Donor )

( rM )$_n$ dN

p( dNp )$_m$ und p( rNp )$_m$

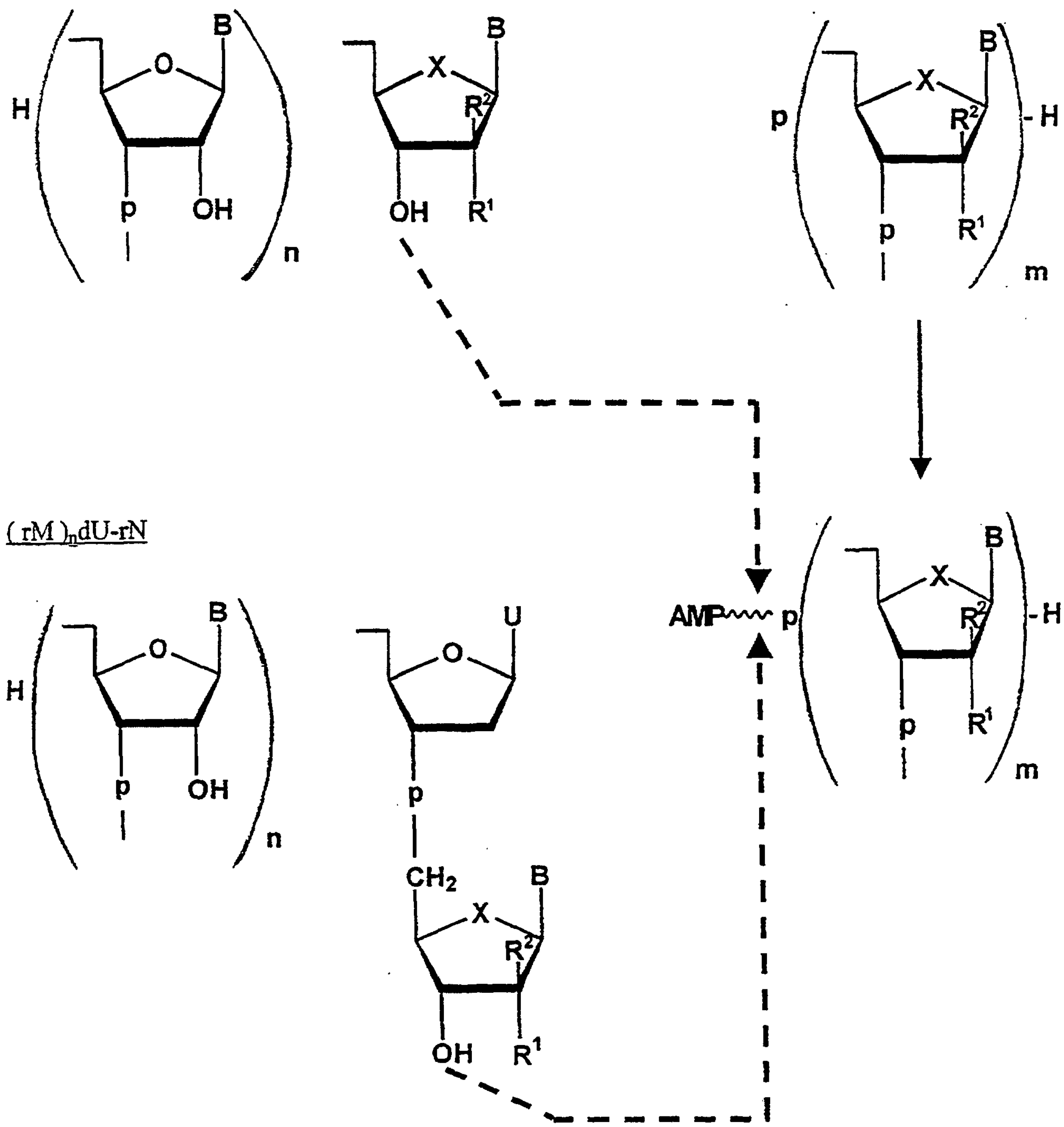

( rM )$_n$dU-rN

Figur 4

3. und 4. Verfahren zur Herstellung von Polymeren

Träger ( Donor )

p( rM )$_n$dNp

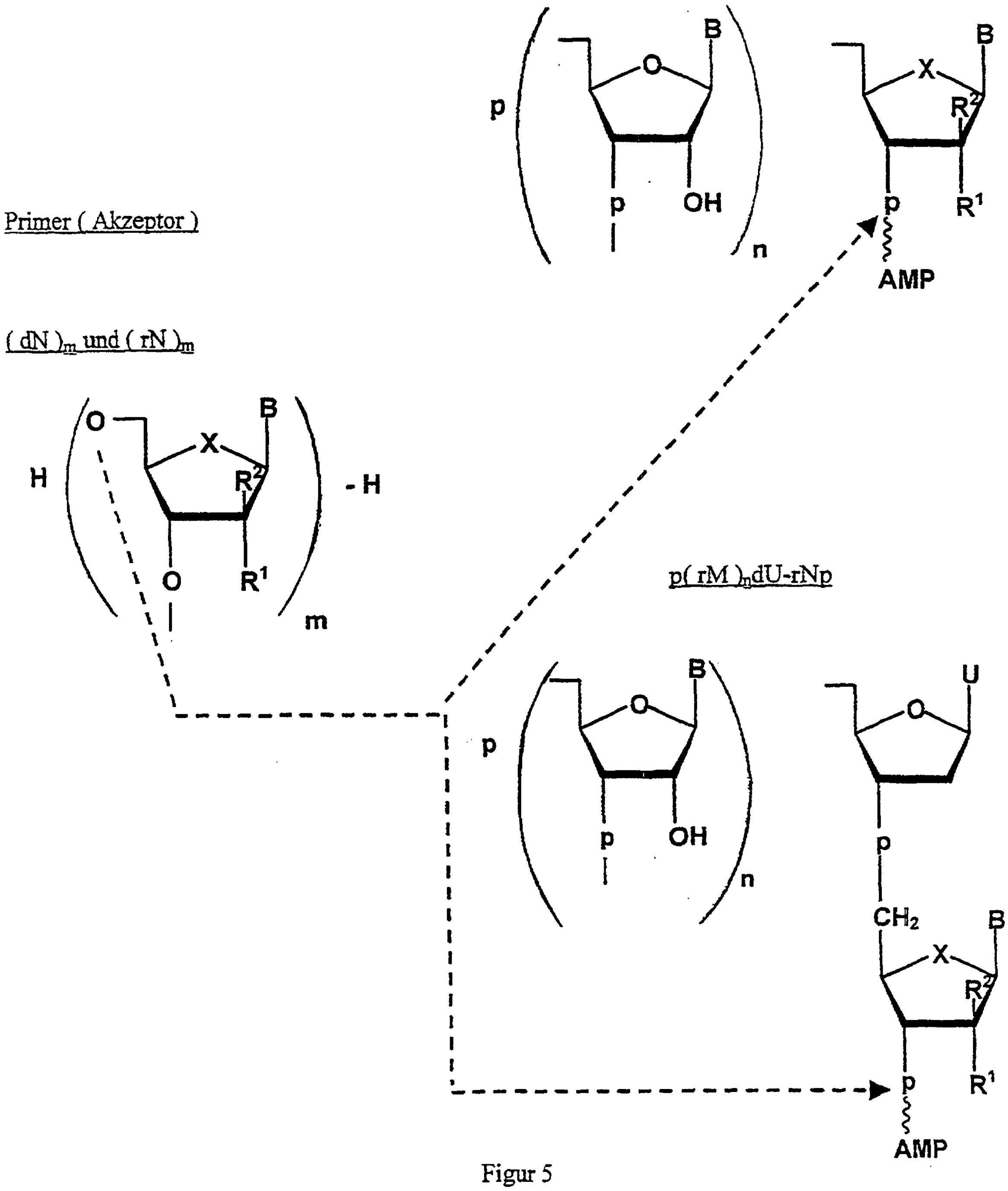

Primer ( Akzeptor )

( dN )$_m$ und ( rN )$_m$

p( rM )$_n$dU-rNp

Figur 5